# NEUE EUROPÄISCHE PATENTSCHRIFT

(11) **EP 2 739 660 B2**
(45) Veröffentlichungstag und Bekanntmachung des Hinweises auf die Entscheidung über den Einspruch: **15.05.2019**
(45) Hinweis auf die Patenterteilung: 29.07.2015
(21) Anmeldenummer: 12784589.9
(22) Anmeldetag: 12.11.2012
(51) Int. Cl.: C08F 220/06, A61L 15/00, C08J 9/28

(54) **SUPERABSORBIERENDE POLYMERE MIT SCHNELLEN ABSORPTIONSEIGENSCHAFTEN SOWIE VERFAHREN ZU DESSEN HERSTELLUNG**
SUPER-ABSORBING POLYMERS WITH RAPID ABSORPTION PROPERTIES AND METHOD FOR PRODUCING THE SAME
POLYMÈRES SUPERABSORBANTS AUX PROPRIÉTÉS D'ABSORPTION RAPIDE AINSI QUE LEUR PROCÉDÉ DE PRODUCTION

(30) Priorität: 17.11.2011 DE 102011086516
(43) Veröffentlichungstag der Anmeldung: 11.06.2014
(73) Patentinhaber: Evonik Degussa GmbH, 45128 Essen (DE)
(72) Erfinder: WATTEBLED, Laurent, 40589 Düsseldorf (DE); HARREN, Jörg, 52499 Baesweiler (DE); NAUMANN, Matthias, 22159 Hamburg (DE); FURNO, Franck, Al-Jasra (BH); LOBERT, Matthias, 45309 Essen (DE); TENI, Rainer, 47447 Moers (DE)
(74) Vertreter: Evonik Patent Association
(86) Internationale Anmeldenummer: PCT/EP2012/072352
(87) Internationale Veröffentlichungsnummer: WO 2013/072268

(56) Entgegenhaltungen:
- EP-A1- 0 644 207
- EP-A1- 0 744 435
- EP-A1- 0 872 491
- EP-A1- 2 399 944
- EP-A1- 2 518 092
- EP-A1- 2 557 095
- EP-A2- 0 827 753
- EP-A2- 0 937 739
- EP-A2- 1 374 919
- WO-A1-2008/046841
- WO-A1-2010/095427
- WO-A1-2011/061315
- WO-A1-2012/156346
- WO-A1-2013/007819
- JP-A- H11 199 602
- US-A- 5 712 316
- US-A- 5 750 585
- US-A- 5 837 789
- US-A1- 2004 214 946
- US-A1- 2005 137 546
- US-A1- 2005 137 546
- US-A1- 2006 182 706
- US-A1- 2009 182 092
- US-A1- 2010 291 389
- Modern Superabsorbent Polymer Technology, ISBN 0-471-19411-5, 1998
- Römpp, Natrium Carbonat, pg. 2823-2824, 1992
- Solvay Soda Light

## Beschreibung

Die vorliegende Erfindung betrifft superabsorbierende Polymere mit schnellen Absorptionseigenschaften sowie Verfahren zu deren Herstellung.

Der aktuelle Trend im Windelaufbau geht dahin, noch dünnere Konstruktionen mit reduziertem Cellulosefaseranteil und erhöhtem Superabsorberanteil herzustellen. Der Vorteil dünnerer Konstruktionen zeigt sich nicht nur in einem verbesserten Tragekomfort, sondern auch in reduzierten Kosten bei Verpackung und Lagerhaltung. Mit dem Trend zu immer dünner werdenden Windelkonstruktionen hat sich das Anforderungsprofil an die Superabsorber deutlich verändert. Von entscheidender Bedeutung ist jetzt die Fähigkeit des Hydrogels zur Flüssigkeitsweiterleitung und -verteilung. Aufgrund der höheren Beladung des Hygieneartikels (Menge an Superabsorber pro Flächeneinheit) darf das Polymer im gequollenen Zustand keine Sperrschicht für nachfolgende Flüssigkeit bilden (Gel-Blocking). Weist das Produkt gute Transporteigenschaften auf, so kann eine optimale Ausnutzung des gesamten Hygieneartikels gewährleistet werden.

Neben der Permeabilität der Superabsorber (SAP) (angegeben in Form der sogenannten "*Saline Flow Conductivity -* SFC") und dem Absorptionsvermögen unter einer Druckbelastung ist insbesondere auch die Absorptionsgeschwindigkeit der Superabsorberpartikel (angegeben in Menge an absorbierter Flüssigkeit pro Gramm Superabsorber pro Sekunde) ein entscheidendes Kriterium, welches Aussagen darüber ermöglicht, ob ein diesen Superabsorber in großer Konzentration enthaltender absorbierender Kern, welcher nur einen geringen Fluffanteil aufweist, in der Lage ist, bei seinem ersten Kontakt mit Flüssigkeiten diese schnell zu absorbieren (sogenannte *"acquisition"*). Diese *"aquisition"* ist bei absorbierenden Kernen mit hohem Superabsorberanteil unter anderem von der Absorptionsgeschwindigkeit des Superabsorbermaterials abhängig.

Aus dem Stand der Technik sind unterschiedliche Schutzrechte bekannt die eine Erhöhung der Absorptionsgeschwindigkeit von Superabsorberpartikel ermöglichen sollen. Aus der WO96/17884A1 ist ein wasserabsorbierendes Harz bekannt in dessen Monomerlösung ein festes Treibmittel mit einem Teilchendurchmesser von 1 bis 100 µm eingesetzt wird. Grundsätzlich werden organische Azoverbindungen bevorzugt und hier im Speziellen Acrylsäuresalze von einer Aminogruppe enthaltende Azoverbindungen. Reine Carbonate, Ammoniumnitrid oder Mischungen hieraus können gegebenenfalls verwendet werden.

Nachteilig ist hierbei der schnelle Umsatz der Azoverbindungen als auch das grundsätzliche Dispergieren der kleinen festen Partikel in der Monomerlösung. Größere Partikel können nicht gut dispergiert werden, ohne dass es zu einer Trennung von Partikeln und Monomerenlösung in der Dispersion vor dem Gelpunkt kommt.

Nachteilig hierbei ist bei der Verwendung von Superaborbern, die aus dem Stand der Technik bekannt sind, dass Leckage-Probleme auftreten, da der SAP entweder die Flüssigkeit zu langsam aufnimmt und/oder über einen ungeeigneten Flüssigkeitstransport verfügt.

Der aktuelle Trend insbesondere im Windelaufbau geht dahin, noch dünnere absorbierende Kerne mit reduziertem Cellulosefaseranteil und erhöhtem Superabsor-beranteil herzustellen. Der Vorteil dünnerer Konstruktionen zeigt sich nicht nur in einem verbesserten Tragekomfort, sondern auch in reduzierten Kosten bei Verpackung und Lagerhaltung. Die neuste Generation von absorbierenden Kernen, die beispielsweise in der WO-A-2008/155722, WO-A-2008/155711, WO-A-2008/155710, WO-A-2008/155702, WO-A-2008/155701, WO-A-2008/155699, EP-A-1 225 857, WO-A-01/15647, WO-A-2011/120504, DE-A-10 2009 049 450, WO-A-2008/117109, WO-A-97/11659, EP-A-0 826 349, WO-A-98/37846, WO-A-95/11653, WO-A-95/11651, WO-A-95/11652, WO-A-95/11654, WO-A-2004/071363 oder WO-A-01/89439 beschrieben wird, ist im wesentlichen Cellulose-frei (weswegen entsprechende Windeln auch als *"fluffless diaper"* bezeichnet werden). Die Immobilisierung der Superabsorberpartikel, die in Cellulose-haltigen absorbierenden Kernen durch die Cellulosefasern erfolgt, kann in dieser neuesten Generation absorbierender Kerne dadurch realisiert werden, dass beispielsweise die Superabsorberpartikel mittels thermoplastischer Fasern auf einer Substratoberfläche immobilisiert werden.

Mit dem Trend zu immer dünner werdenden Windelkonstruktionen und dem Wegfall der temporären Flüssigkeitsspeicherungs- und Weiterleitungsfunktion der Cellulosefasern hat sich das Anforderungsprofil an die Superabsorber deutlich verändert. Von entscheidender Bedeutung ist jetzt die Fähigkeit des Hydrogels, das Auslaufen (Leakage) von Urin direkt bei der Miktion zu verhindern. Dies wird durch die Eigenschaft des Superabsorbers/Hydrogels erreicht, die Flüssigkeit während der Quellung effektiv aufzunehmen und in der Gelschicht zu verteilen, bei gleichzeitiger Minimierung der Menge ungebundenen Urins in der Windel. Vorteilhafte Superabsorber führen aufgrund von guten Transporteigenschaften auch zu einer optimalen Ausnutzung des gesamten Hygieneartikels.

Aus der US 5,154,713 sind wasserabsorbierende Polymere bekannt die mittels eines Carbonat-Treibmittels in der Monomerlösung hergestellt werden. Hierbei erfolgt das Einbringen der Carbonatpartikel in die Monomerenlösung deutlich vor der eigentlichen Polymerisation, und der Initiatorzugabe von 5 bis 15 Minuten nach dem Dispergieren des Carbonat-Treibmittels, wodurch eine gleichmäßige Verteilung dieser Carbonatpartikel nicht mehr gewährleistet ist und ein nicht unerheblicher Teil des Carbonats gegebenenfalls schon wieder ausgetragen ist.

Aus der EP0644207 sind superabsorbierende Polymere bekannt die ebenfalls mit einem organischen Carbonattreibmittel in der Monomerlösung versetzt werden. Nachteilig ist hier die Verwendung von Aminverbindungen als auch der Verbleib der Abspaltprodukte der organischen Carbonate im Superabsorber.

Aus der WO 2010/095427 sind wasserabsorbierende Polymere bekannt, in denen in die Monomerlösung ein Gas dispergiert wird. Hierbei handelt es sich um Stickstoff, Argon, Helium, Kohlendioxid oder ähnliche, wodurch eine porösere Struktur gewährleistet werden soll. Diese Mikrobläschen sollen mittels Polyoxyethylen-(20)-Sorbitanmonostearat solange in der Monomerlösung gehalten werden bis die Polymerisation einsetzt. Nachteilig ist hierbei, dass die Tenside wieder aus dem Endprodukt ausgewaschen werden können und die Perfomance negativ beeinflußen.

Von entscheidender Bedeutung ist jetzt die Fähigkeit des Hydrogels, das Auslaufen (Leakage) von Urin direkt bei der Miktion zu verhindern. Dies wird durch die Eigenschaft des Superabsorbers/Hydrogels erreicht, die Flüssigkeit während der Quellung effektiv aufzunehmen und in der Gelschicht zu verteilen, bei gleichzeitiger Minimierung der Menge ungebundenen Urins in der Windel. Vorteilhafte Superabsorber führen aufgrund von guten Transporteigenschaften auch zu einer optimalen Ausnutzung des gesamten Hygieneartikels.

Unter dem Begriff "Rewet" wird im Allgemeinen die Eigenschaft eines Superabsobers oder eines Verbundes der einen Superabsorber enthält, verstanden, Unter Druckbelastung an eine saugfähige Lage Flüssigkeit abzugeben. Unter dem Begriff "saugfähige Lage" werden z. B. Papier, Filterpapier, Collagen, Schwämme. Schäume oder Ähnliche verstanden.

Aus der EP1858998B1 sind superabsorbierende Schäume bekannt, bei denen die Monomerlösung nur unter erhöhtem Druck von 12 bar durch Zugabe von Kohlendioxid und Tensiden einen Schaum ergibt.

Die bisher aus dem Stand der Technik bekannten Superabsorber sind jedoch nur unzureichend für einen Einsatz in der vorstehend beschriebenen neuen Generation von Cellulose-freien Windelkonstruktionen geeignet.

Allgemein liegt der vorliegenden Erfindung die Aufgabe zu Grunde, die sich aus dem Stand der Technik ergebenden Nachteile zu überwinden.

Es ist insbesondere die Aufgabe der vorliegenden Erfindung, ein Verfahren zur Herstellung eines wasserabsorbierenden Polymeren zur Verfügung zu stellen, das eine verbesserte Quellgeschwindigkeit und schnellere Aufnahme von Flüssigkeiten aufweist unter gleichzeitiger Beibehaltung der Gesamtqualität und insbesondere einer hohen Permeabilität.

Außerdem ist es eine weitere Aufgabe das Verfahren in ökonomisch einfacher Weise durchzuführen, wobei die Verwendung von organischen Additiven minimiert wird, als auch eine drucklose Arbeitsweise gewährleistet werden soll.

Es ist insbesondere eine Aufgabe der vorliegenden Erfindung, ein Verfahren bereitzustellen, nach dem wasserabsorbierende Polymere hergestellt werden können, wobei eine besonders hohe Quellgeschwindigkeit gewährleistet werden kann.

Eine weitere Aufgabe der vorliegenden Erfindung besteht zusätzlich darin, ein Verfahren bereit zu stellen, nach dem wasserabsorbierende Polymere hergestellt werden können die einen schnellen und aktiven Flüssigkeitstransport, z.B. in dünnen Windeln gewährleisten, so dass eine schnelle Aufnahme und eine gute Verteilung, d. h. eine entsprechende Kapillarität gewährleistet wird.

Eine weitere Aufgabe der Erfindung besteht vornehmlich darin, ein wasserabsorbierendes Polymer, Verbunde, die derartige wasserabsorbierende Polymere enthalten, und chemische Produkte, die derartige wasserabsorbierende Polymere oder Verbunde enthalten, anzugeben, wobei die wasserabsorbierenden Polymeren eine erhöhte Absorptionsgeschwindigkeit für wässrige Lösungen aufweisen.

Diese Aufgaben werden gelöst durch den Gegenstand der kategoriebildenden Ansprüche. Vorteilhafte Ausgestaltungen und Weiterbildungen, die einzeln oder in Kombination auftreten können, sind Gegenstand der jeweils abhängigen Ansprüche.

Ein Beitrag zur Lösung der eingangs genannten Aufgabe leistet das Verfahren zur Herstellung eines wasserabsorbierendes Polymers mit den Merkmalen des Anspruch 1.
(α4) 0 bis 30 Gew.-%, bevorzugt 1 bis 20 Gew.-% und besonders bevorzugt 5 bis 10 Gew.-% wasserlöslichen Polymeren,
(α5) 0 bis 20 Gew.-%, bevorzugt 0,01 bis 7 Gew.-% und besonders bevorzugt 0,05 bis 5 Gew.-% eines oder mehrerer Hilfsstoffe, wobei die Summe der Gewichtsmengen (α1) bis (α5) 100 Gew.-%,

(ii) radikalische Polymerisation unter Vernetzung, um ein wasserunlösliches, wässriges unbehandeltes Hydrogel-Polymer zu bilden,
(iii) Trocknen des Hydrogel-Polymers,
(iv) Mahlen und Absieben des Hydrogel -Polymers,
(V) Oberflächennachvernetzung des gemahlenen und gesiebten Hydrogel-Polymers und
(vi) Trocknung und Konfektionierung des wasserabsorbierenden Polymers,
wobei
der wässrigen Monomerlösung, vor der Zugabe des Initiators und dem Start der radikalischen Polymerisation, 0,01 bis 5 Gew.-%, bevorzugt 0,02 bis 2 Gew.-% und besonders bevorzugt 0,07 bis 1 Gew.-% mindestens eines Tensides aus der Gruppe der nicht ionischen, ionischen oder amphoteren Tenside und gegebenenfalls 0,01 bis 5 Gew.-%, bevorzugt 0,02 bis 2 Gew. % und besonders bevorzugt 0,07 bis 1 Gew.-% eines Blähmittels mit einer Partikelgröße von 10 µm bis 900µm zugesetzt werden, bezogen auf das wasserabsorbierende Polymere.

Unter dem Begriff "wasserabsorbierendes Polymer" wird erfindungsgemäß der Superabsorber verstanden.

In einer weiteren Ausführungsform wird ein Hydrogel, umfassend die Verfahrenschritte
(i) Mischen von
   (α1) 0,1 bis 99,999 Gew.-%, bevorzugt 20 bis 98,99 Gew.-% und besonders bevorzugt 30 bis 98,95 Gew.-% polymerisierbaren, ethylenisch ungesättigten, säuregruppenhaltigen Monomeren oder deren Salze oder polymerisierbaren, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
   (α2) 0 bis 70 Gew.-%, bevorzugt 1 bis 60 Gew.-% und besonders bevorzugt 1 bis 40 Gew.-% polymerisierbaren, ethylenisch ungesättigten, mit (α1) copolymerisierbaren Monomeren,
   (α3) 0,001 bis 10 Gew.-%, bevorzugt 0,01 bis 7 Gew.-% und besonders bevorzugt 0,05 bis 5 Gew.-% eines oder mehrerer Vernetzer,
   (α4) 0 bis 30 Gew.-%, bevorzugt 1 bis 20 Gew.-% und besonders bevorzugt 5 bis 10 Gew.-% wasserlöslichen Polymeren,
   (α5) 0 bis 20 Gew.-%, bevorzugt 0,01 bis 7 Gew.-% und besonders bevorzugt 0,05 bis 5 Gew.-% eines oder mehrerer Hilfsstoffe, wobei die Summe der Gewichtsmengen (α1) bis (α5) 100 Gew.-%,
(ii) radikalische Polymerisation unter Vernetzung, um ein wasserunlösliches, wässriges unbehandeltes Hydrogel-Polymer zu bilden,
(iii) Trocknen des Hydrogel-Polymers,
(iv) Mahlen und Absieben des Hydrogel-Polymers,
wobei
der wässrigen Monomerlösung, vor der Zugabe des Initiators und dem Start der radikalischen Polymerisation, 0,01 bis 5 Gew.-%, bevorzugt 0,02 bis 2 Gew.-% und besonders bevorzugt 0,07 bis 1 Gew.-% mindestens eines Tensides aus der Gruppe der nicht ionischen, ionischen oder amphoteren Tenside und gegebenenfalls 0,01 bis 5 Gew.-%, bevorzugt 0,02 bis 2 Gew. % und besonders bevorzugt 0,07 bis 1 Gew.-% eines Blähmittels mit einer Partikelgröße von 10 µm bis 900µm zugesetzt werden, bezogen auf das Hydrogel-. Polymere.

Bevorzugt wird erfindungsgemäß gewährleistet, dass die Tenside in das Polymernetzwerk einpolymerisiert werden. Vorteilhafterweise wird hierdurch die Menge an extrahierbaren tensidischen Bestandteilen stark erniedrigt und die Oberflächenspannung entsprechend nur minimal reduziert.

Erfindungsgemäß wird durch die Zugabe von Tensiden und Blähmittel zu der Monomeren Lösung nach Polymerisation eine fein-poröse Gel-Struktur erzielt und werden Superabsorber-Pulver erhalten, die eine größere Oberfläche aufweisen. Vorteilhafterweise wird durch die erfindungsgemäße Vergrößerung der gesamten Oberfläche eine schnellere Absorption der Flüssigkeit, im Vergleich zu herkömmlichen SAPs gewährleistet. Dies wird durch den sogenannten FSR-Wert dargestellt. Die erfindungsgemäßen wasserabsorbierenden Polymere haben einen FSR im Bereich von 0,3 bis 0,65 bevorzugt 0,35 bis 0,60. Erfindungsgemäß ist es besonders bevorzugt, wenn der FSR-Wert größer als 0,40 g/g/s ist.

Erfindungsgemäß erfolgt trotz des Einsatzes der Tenside keine Erniedrigung der Oberflächenspannungswerte der Hydrogel-Polymere oder Superabsorber.

Erfindungsgemäß liegt die Permeabilität, der als SFC-Wert bezeichnet wird (in der vorliegenden Erfindung immer bezogen auf 1,5g), der wasserabsorbierenden Polymerzusammensetzung im Bereich von 30 bis 200, bevorzugt 50 bis 180 und besonders bevorzugt im Bereich von 70 bis 150.

Erfindungsgemäß liegt die Oberflächenspannung im Bereich von über 50 mN/m, bevorzugt über 55 mN/m, besonders bevorzugt über 60 mN/m und am meisten bevorzugt über 62 mN/m. Die Oberflächenspannung ist erfindungsgemäß nicht größer als 68 mN/m..

Die Partikelverteilung PSD des Hydrogel-Polymers ist erfindungsgemäß so ausgebildet, dass mehr als 60 Gew. % der Partikel im Bereich von 300 µm bis 600µm liegen und weniger als 5 Gew.% der Partikel kleiner 150 µm sind.

Bei einer niedrigen Oberflächenspannung, führt dies im Allgemeinen zu erhöhten "Rewet" Werten, z.B. Backsheet Rewet oder zu Leakage für die Windel in denen solche Superabsorber eingesetzt werden. Vorteilhafterweise wird dieses Problem durch das erfindungsgemäße Verfahren und in Form der einpolymerisierbaren Tenside unterbunden. Zusätzlich, zeigen die Superabsorber vergleichbare Performance hinsichtlich Retention (CRC), Absorption gegen Druck (AAP).

Massgeschneiderte Tenside werden für die Herstellung der erfindungsgemäßen Superabsorber zu der Monomeren Lösung zugesetzt. Diese speziellen "surfactants" enthalten funktionelle Gruppen, die polymerisierbar sind. Bevorzugte chemische Strukturen sind:

R¹-(EO)ₙ-block-(PO)ₘ-R²

R¹ oder R² = Methyl, Ethyl, n-Propyl, iso-Propyl, n-Butyl, iso-Butyl, n-Pentyl, 2 methyl-Bütyl, 2,2-Dimethylpropyl, -OH , Acetyl oder Allyl ist und n = 2 bis 20 und m = 2 bis 20 bedeutet EO und PO sind respektiv hydrophile und hydrophobe Blöcke, um tensidische Eigenschaften zu generieren. Reaktive Allyl-Gruppen werden bevorzugt, da sie erfindungsgemäß später als Acryl-Gruppen einpolymerisieren und stabiler in der Hydrolyse sind.

Besonders bevorzugt ist es, wenn R¹ oder R² einem Allyl-Rest und der andere Rest einem Acetyl-, oder -OH- Rest entspricht.

Im Rahmen der vorliegenden Erfindung wird unter dem Begriff "EO" ein Ether, der durch Polyaddition von Ethylenoxid gebildet und als Polyether oder Polyethylenglykole bezeichnet wird, verstanden. In der vorliegenden Anmeldung handelt es sich bei dem Begriff "Allyl-(EO)ₙ-", um eine Gruppe der Formel CH₂=CH-CH₂-O-(CH2-CH₂-O)ₙ-. Im Besonderen handelt es sich um Verbindungen bei denen n für eine ganze Zahl zwischen 2 bis 20 entspricht. Bevorzugt sind Verbindungen in den n gleich 4 bis 12 und besonders bevorzugt n gleich 5 bis 8 entspricht.

Im Rahmen der vorliegenden Erfindung wird unter dem Begriff "PO" grundsätzlich Propylenoxid gemäß der Formel -(CH₂- CH(CH₃)-O)ₘ verstanden. Hierbei steht m für eine ganze Zahl von 2 bis 20. Bevorzugt sind Verbindungen in den m 3 bis 12 und besonders bevorzugt m 4 bis 7 entspricht.

Bevorzugt für die Verteilung der beiden unterschiedlichen Blöcke ist 2≤ m≤n≤20 so dass ein hydrophiler Grundkörper gewährleistet ist und das Tensid eine definierte Wasserlöslichkeit besitzt.

Erfindungsgemäß sind Tenside der folgenden Formel:

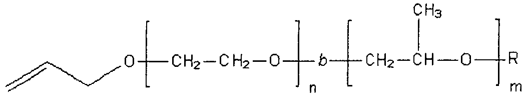

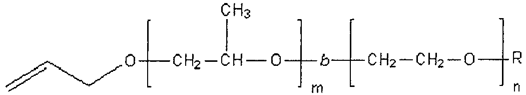

Hierbei entspricht R gleich R¹ oder R².

Im Rahmen der vorliegenden Erfindung kann es sich bei den einzelnen PO-Einheiten um isotaktische, syndiotaktische oder ataktische Abfolgen der Konfiguration in dem Molekül handeln.

Als Blähmittel können alle Carbonate aus der Gruppe von Lithium-, Natrium-, Kalium-, Rubidium-, Cäsiumcarbonat, oder höherwertige Metallionen wie Beryllium-, Calcium-, Magnesium-, Strontiumcarbonat oder Mischungen dieser verwendet werden. Als weitere Verbindungen können auch granulierte Carbonate eingesetzt werden, die auch als Mischsalze aus einem Carbonat und/oder Percarbonat mit einem weiteren als Deckschicht fungierenden Salz wie z. B. einer Sulfatverbindung hergestellt werden. Erfindungsgemäß weisen die Blähmittel eine Partikelgröße von 10 µm bis 900 µm, bevorzugt 50 µm bis 500 µm und besonders bevorzugt von 100 µm bis 450 µm auf.

Erfindungsgemäß werden die Tenside im Allgemeinen zeitgleich mit dem Vernetzer eingesetzt. Das Blähmittel wird in einer Ausführungsform nach der Zugabe des Tensides zugegeben. In weiteren Ausführungsformen kann das Blähmittel zeitgleich oder vor dem Tensid zugeführt werden.

Bei der Zugabe von Blähmitteln bzw. Soda entstehen Bläschen, die in Anwesenheit von Tensiden einen kleineren Durchmesser aufweisen

Erfindungsgemäß stabilisieren die Tenside die große Gasoberfläche, die in der Lösung durch das Blähmittel entsteht. Durch die parallel ablaufende Polymerisation wird eine fein porige Struktur fixiert (poröses Gel). Die Tenside werden während der Polymerisation "inaktiviert" d.h. sie können (sich) im Polymer Netzwerk einbauen bzw. werden aufgrund ihrer reaktiven Funktionalität eingebaut.

Die beobachtete Synergie zwischen Tensiden und Blähmitteln erlaubt vorteilhafterweise den Einsatz von geringeren Mengen an Blähmittel wie z.B. Carbonat. Typische Nachteile, die mit Carbonaten verbunden werden, sind u.a. die möglichen Schwierigkeiten bei der Mischung und Zugabe in der Lösung, sowie eine unkontrollierte Dispersion der entstehenden Bläschen wie z.B. eine starke Koaleszenz oder zu große Blasen oder der Verlust an anderen Eigenschaften des SAPs.

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 10 Mol-%, besonders bevorzugt zu mindestens 25 bis 50 Mol-% und darüber hinaus bevorzugt zu 50-90 Mol-% neutralisiert. Die Neutralisation der Monomere (α1) kann vor, aber auch nach der Polymerisation erfolgen. Hierbei erfolgt die Teilneutralisierung zu mindestens 10 Mol-%, besonders bevorzugt zu mindestens 25 bis 50 Mol-% und darüber hinaus bevorzugt zu 50-90 Mol-%. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als "*Mixed-Bed Ion-Exchange Absorbent Polymers*" (MBIEA-Polymere) bezeichnet und sind unter anderem in der WO 99/34843 offenbart. Die Offenbarung der WO 99/34843 wird hiermit als Referenz eingeführt und gilt somit als Teil der Offenbarung. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Monoethylenisch ungesättigte, säuregruppenhaltige Monomere (α1) sind Acrylsäure.

Neben diesen carboxylatgruppenhaltigen Monomeren sind als monoethylenisch ungesättigte, säuregruppenhaltige Monomere (α1) des Weiteren ethylenisch ungesättigte Sulfonsäuremonomere oder ethylenisch ungesättigte Phosphonsäuremonomere bevorzugt, sofern Hydrogele nach Anspruch 2 betroffen sind.

Als ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Stryrolsulfonsäure bevorzugt. Als Acryl- bzw. Methacrylsulfonsäuren sind Sulfoethyl(meth)acrylat, Sulfopropyl(meth)acrylat, 2-Hydroxy-3-methacryloxypropylsulfonsäure sowie (Meth)Acrylamidoalkylsulfonsäuren wie 2-Acrylamido-2-methylpropansulfonsäure bevorzugt.

Als ethylenisch ungesättigte Phosphonsäuremonomere sind Vinylphosphonsäure, Allylphosphonsäure, Vinylbenzylphosponsäure, (Meth)acrylamidoalkylphosphonsäuren, Acrylamidoalkyldiphosphonsäuren, phosponomethylierte Vinylamine und (Meth)acryl-phosphonsäurederivate bevorzugt.

Als monoethylenisch ungesättigte, mit (α1) copolymerisierbare Monomere (α2) sind Acrylamide und Methacrylamide bevorzugt.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino(meth)acrylamid, Dimethyl(meth)acrylamid oder Diethyl(meth)acrylamid Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäurester und Methacrylsäurester, wie Methyl(meth)acrylat, Ethyl(meth)acrylat, Propyl(meth)acrylat oder Butyl(meth)acrylat, sowie Vinylacetat, Styrol und Isobutylen bevorzugt.

Erfindungsgemäß bevorzugte Vernetzer (α3) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (α1) oder (α2) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV). Dabei wird durch die Verbindungen der Vernetzerklasse I eine Vernetzung der Polymere durch die radikalische Polymerisation der ethylenisch ungesättigten Gruppen des Vernetzermoleküls mit den monoethylenisch ungesättigten Monomeren (α1) oder (α2) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere (α1) oder (α2) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren (α1) oder (α2).

Bevorzugte Verbindungen der Vernetzerklasse I sind Poly(meth)acrylsäureester, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth)allyverbindungen, (Meth)acrylsäureester einer Monovinylverbindung oder (Meth)acrylsäureester einer Mono(meth)allyl- verbindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen. Die Offenbarungen werden hiermit als Referenz eingeführt und gelten somit als Teil der Offenbarung.

Als Verbindungen der Vernetzerklasse I seien als Beispiel genannt Alkenyldi(meth)acrylate, beispielsweise Ethylenglykoldi(meth)acrylat, 1,3-Propylenglykoldi(meth)acrylat, 1,4-Butylenglykoldi(meth)acrylat, 1,3-Butylenglykoldi(meth)acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopentandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'-(1,2-Di-hydroxyethylen)bis(meth)acrylamid, N,N'-Hexamethylenbis(meth)acryl-acrylamid oder N,N'-Methylenbis(meth)acrylamid, Polyalkoxydi(meth)acrylate, beispielsweise Diethylenglykoldi(meth)acrylat, Triethylenglykoldi(meth)acrylat, Tetraethylenglykoldi(meth)acrylat, Dipropylenglykoldi(meth)acrylat, Tripropylenglykoldi(meth)acrylat oder Tetrapropylenglykoldi-(meth)acrylat, Bisphenol-A-di(meth)acrylat, ethoxyliertes Bisphenol-A-di(meth)acrylat, Benzylidindi(meth)acrylat, 1,3-Di(meth)acryloyloxy-propanol-2, Hydrochinondi(meth)acry- lat, Di(meth)acrylatester des vorzugsweise mit 1 bis 30 Mol Alkylenoxid pro Hydroxylgruppe oxyalkylierten, vorzugsweise ethoxylierten Trimethylolpropans, Thioethylenglykoldi(meth)-acrylat, Thiopropylenglykoldi(meth)acrylat, Thiopolyethylenglykoldi(meth)acrylat, Thiopolypropylenglykoldi(meth)acrylat, Divinylether, beispielsweise 1,4-Butandioldivinylether, Divinylester, beispielsweise Divinyladipat, Alkandiene, beispielsweise Butadien oder 1,6-Hexadien, Divinylbenzol, Di(meth)allylverbindungen, beispielsweise Di(meth)allylphthalat oder Di(meth)allylsuccinat, Homo- und Copolymere von Di(meth)allyldimethylammoniumchlorid und Homo- und Copolymere von Diethyl(meth)allyl- aminomethyl(meth)acrylat-ammoniumchlorid, Vinyl-(meth)acryl-Verbindungen, beispielsweise Vinyl(meth)acrylat, (Meth)allyl-(meth)acryl-Verbindungen, beispielsweise (Meth)allyl(meth)acrylat, mit 1 bis 30 Mol Ethylenoxid pro Hydroxylgruppe ethoxyliertes (Meth)allyl(meth)acrylat, Di(meth)allylester von Polycarbonsäuren, beispielsweise Di(meth)allylmaleat, Di(meth)allyfumarat, Di(meth)allylsuccinat oder Di(meth)allylterephthalat, Verbindungen mit 3 oder mehr ethylenisch ungesättigten, radikalisch polymerisierbaren Gruppen wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi(meth)acrylat, 3-Allyloxy-1,2-propandioldi(meth)acrylat, Tri- (meth)allylcyan-urat, Tri(meth)allylisocyanurat, Pentaerythrittetra(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri(meth)allylamin, Di(meth)allylalkylamine, beispielsweise Di(meth)allylmethylamin, Tri-(meth)allylphosphat, Tetra(meth)allylethylendiamin, Poly(meth)allylester, Tetra(meth)allyloxiethan oder Tetra(meth)allylammoniumhalide.

Als Verbindung der Vernetzerklasse II sind Verbindungen bevorzugt, die mindestens zwei funktionelle Gruppen aufweisen, die in einer Kondensationsreaktion (=Kondensaionsvernetzer), in einer Additionsreaktion oder in einer Ringöffnungsreaktion mit den funktionellen Gruppen der Monomere (α1) oder (α2), bevorzugt mit Säuregruppen, der Monomeren (α1), reagieren können. Bei diesen funktionellen Gruppen der Verbindungen der Vernetzerklasse II handelt es sich vorzugsweise um Alkohol-, Amin-, Aldehyd-, Glycidyl-, Isocyanat-, Carbonat- oder Epichlorfunktionen.

Als Verbindung der Vernetzerklasse II seien als Beispiele genannt Polyole, beispielsweise Ethylenglykol, Polethylenglykole wie Diethylenglykol, Triethylenglykol und Tetraethylenglykol, Propylenglykol, Polypropylenglykole wie Dipropylenglykol, Tripropylenglykol öder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriaamin, Triethylentetraamin, Tetraethylenpentaamin oder Pentaethylenhexaamin, Polyglycidylether-Verbindungen wie Ethylenglykoliglycidylether, Polyethylenglykoldiglycidylether, Glycerindiglycidylether, Glycerinpolyglycidyether, Pentareritritpolyglycidylether, Propylenglykoldiglycidylether Polypropylenglykoldiglycidylether, Neopentylglykoldiglycidylether, Hexandiolglycidylether, Trimethylolpropanpolyglycidylether, Sorbitolpolyglycidylether, Phtahlsäurediglycidylester, Adipinsäurediglycidylether, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbutanol-tris[3-(1-aziridinyl)propionat], 1,6-Hexamethylendiethylenharnstoff und Diphenylmethan-bis-4,4'-N,N'-diethylenharnstoff, Halogenperoxide beispielsweise Epichlor- und Epibromhydrin und α-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, P.oly-1,3-dioxolan-2-on, polyquartäre Amine wie Kondensationsprodukte von Dimethylaminen und Epichlorhydrin. Als Verbindungen der Vernetzerklasse II sind des Weiteren Polyoxazoline wie 1,2-Ethylenbisoxazolin, Vernetzer mit Silangruppen wie γ-Glycidoxypropyltrimethoxysilan und γ-Aminopropyltrimethoxysilan, Oxazolidinone wie 2-Oxazolidinon, Bis- und Poly-2-oxazolidinone und Diglykolsilikate bevorzugt.

Als Verbindungen der Klasse III sind hydroxyl- oder aminogruppenhaltige Ester der (Meth)acrylsäure, wie beispielsweise 2-Hydroxyethyl(meth)acrylat und 2-Hydroxypropyl-(meth)acrylat sowie hydroxyl- oder aminogruppenhaltige (Meth)acrylamide oder Mono(meth)allylverbindungen von Diolen bevorzugt.

Die polyvalenten Metallkationen der Vernetzerklasse IV leiten sich vorzugsweise von ein- oder mehrwertigen Kationen ab, die einwertigen insbesondere von Alkalimetallen, wie Kalium, Natrium, Lithium, wobei Lithium bevorzugt wird. Bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. AlCl₃ × 6H₂O, NaAl(SO₄)₂ × 12 H₂O, KAl(SO₄)₂ × 12 H₂O oder Al₂(SO₄)₃×14-18 H₂O eingesetzt. Besonders bevorzugt werden Al₂(SO₄)₃ und seine Hydrate als Vernetzer der Vernetzungsklasse IV verwendet.

Die im erfindungsgemäßen Verfahren eingesetzten Superabsorberteilchen sind vorzugsweise durch Vernetzer der folgenden Vernetzerklassen bzw. durch Vernetzer der folgenden Kombinationen von Vernetzerklassen vernetzt: I, II, III, IV, III, I III, I IV, I II III, I II IV, I III IV, II III IV, II IV oder III IV. Die vorstehenden Kombinationen von Vernetzerklassen stellen jeweils eine bevorzugte Ausführungsform von Vernetzern eines im erfindungsgemäßen Verfahren eingesetzten Superabsorberteilchens dar.

Weitere bevorzugte Ausführungsformen der im erfindungsgemäßen Verfahren eingesetzten Superabsorberteilchen sind Polymere, die durch einen beliebigen der vorstehend genannten Vernetzer der Vernetzerklasse I vernetzt sind. Unter diesen sind wasserlösliche Vernetzer bevorzugt. In diesem Zusammenhang sind N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den Superabsorberteilchen wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsstoffe (α5) sind in den Polymeren, organische oder anorganische Partikel wie beispielsweise Geruchsbinder, insbesondere Zeolithe oder Cyclodextrine, Hautpflegesubstanzen, oberflächenaktive Mittel oder Antioxidantien enthalten.

Zu den bevorzugten organischen Hilfsstoffen gehören Cyclodextrine oder deren Derivate sowie Polysaccharide. Außerdem sind Cellulose und Cellulosederivate wie CMC, Celluloseether bevorzugt. Als Cyclodextrine oder Cyclodextrinderivate sind dabei diejenigen Verbindungen bevorzugt, die in DE-A-198 25 486 auf der Seite 3, Zeile 51 bis Seite 4, Zeile 61 offenbart sind. Der vorstehend genannte Abschnitt dieser veröffentlichten Patentanmeldung wird hiermit als Referenz eingeführt und gilt als Teil der Offenbarung der vorliegenden Erfindung. Besonders bevorzugte Cyclodextrine sind nicht derivatisierte α-, β-, γ- oder δ-Cyclodextrine.

Als anorganische partikuläre Hilfsstoffe können alle Materialen eingesetzt werden, welche üblicherweise zur Modifizierung der Eigenschaften wasserabsorbierender Polymere eingesetzt werden. Zu den bevorzugten anorganischen Hilfsstoffen gehören Sulfate wie Na₂SO₄, Lactate wie etwa Natriumlactat, Silikate, insbesondere Gerüstsilikate wie Zeolithe oder Silikate, die durch Trocknung wässriger Kieselsäurelösungen oder Kieselsolen erhalten wurden, beispielsweise die kommerziell erhältlichen Produkte wie Fällungskieselsäuren und pyrogene Kieselsäuren, beispielsweise Aerosile mit einer Teilchengröße im Bereich von 5 bis 50 nm, vorzugsweise im Bereich von 8 bis 20 nm wie "Aerosil 200" der Evonik Industries AG, Aluminate, Titandioxide, Zinkoxide, Tonmaterialien und weitere dem Fachmann geläufige Mineralien sowie kohlenstoffhaltige anorganische Materialien.

Bevorzugte Silikate sind alle natürlichen oder synthetischen Silikate, die in Hollemann und Wiberg, Lehrbuch der Anorganischen Chemie, Walter de Gruyter-Verlag, 91.-100. Auflage, 1985 auf den Seiten 750 bis 783, als Silikate offenbart sind. Der vorstehend genannte Abschnitt dieses Lehrbuchs wird hiermit als Referenz eingeführt und gilt als Teil der Offenbarung der vorliegenden Erfindung.

Besonders bevorzugte Silikate sind die Zeolithe. Als Zeolithe können alle dem Fachmann bekannten synthetischen oder natürlichen Zeolithe eingesetzt werden. Bevorzugte natürliche Zeolithe sind Zeolithe aus der Natrolith-Gruppe, der Harmoton-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabasit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmoton, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS®.

Als Zeolithe können Zeolithe des so genannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie β-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Als Aluminate werden vorzugsweise die in der Natur vorkommenden Spinelle, insbesondere gewöhnlicher Spinell, Zinkspinell, Eisenspinell oder Chromspinell eingesetzt.

Bevorzugtes Titandioxid ist das reine Titandioxid in den Kristallformen Rutil, Anatas und Brookit, sowie eisenhaltige Titandioxide wie beispielsweise Ilmenit, calciumhaltige Titandioxide wie Titanit oder Perowskit.

Bevorzugte Tonmaterialien sind diejenigen, die in Hollemann und Wiberg, Lehrbuch der Anorganischen Chemie, Walter de Gruyter-Verlag, 91.-100. Auflage, 1985 auf den Seiten 783 bis 785, als Tonmaterialien offenbart sind. Besonders Der vorstehend genannte Abschnitt dieses Lehrbuchs wird hiermit als Referenz eingeführt und gilt als Teil der Offenbarung der vorliegenden Erfindung. Besonders bevorzugte Tonmaterialien sind Kaolinit, Illit, Halloysit, Montmorillonit sowie Talk.

Weiterhin sind als anorganische Feinteilchen die Metallsalze der Mono-, Oligo- und Polyphosphorsäuren erfindungsgemäß bevorzugt. Hierunter sind insbesondere die Hydrate bevorzugt, wobei die Mono- bis Deca-Hydrate und Tri-Hydrate besonders bevorzugt sind. Als Metalle kommen insbesondere Alkali- und Erdalkalimetalle in Betracht, wobei die Erdalkalimetalle bevorzugt sind. Hierunter sind Mg und Ca bevorzugt und Mg besonders bevorzugt.. Im Zusammenhang mit Phosphaten, Phosphorsäuren und deren Metallverbindungen wird auf Hollemann und Wiberg, Lehrbuch der Anorganischen Chemie, Walter de Gruyter-Verlag, 91.-100. Auflage, 1985, auf den Seiten 651 bis 669 verwiesen. Der vorstehend genannte Abschnitt dieses Lehrbuchs wird hiermit als Referenz eingeführt und gilt als Teil der Offenbarung der vorliegenden Erfindung.

Bevorzugte kohlenstoffhaltige, jedoch nicht organische Hilfsstoffe sind diejenigen reinen Kohlenstoffe, die in Hollemann und Wiberg, Lehrbuch der Anorganischen Chemie, Walter de Gruyter-Verlag, 91.-100. Auflage, 1985 auf den Seiten 705 bis 708 als Graphite genannt sind. Der vorstehend genannte Abschnitt dieses Lehrbuchs wird hiermit als Referenz eingeführt und gilt als Teil der Offenbarung der vorliegenden Erfindung. Besonders bevorzugte Graphite sind künstliche Graphite wie beispielsweise Koks, Pyrographit, Aktivkohle oder Ruß.

Die im erfindungsgemäßen Verfahren erhaltenen wasserabsorbierende Polymere sind vorzugsweise dadurch erhältlich, dass zunächst aus den vorgenannten Monomeren und Vernetzern ein Hydrogel-Polymer (VP) in partikulärer Form hergestellt wird. Die Herstellung dieses Ausgangsmaterials für die wasserabsorbierenden Polymere erfolgt beispielsweise durch Massepolymerisation, die vorzugsweise in Knetreaktoren wie Extrudern erfolgt, Lösungspolymerisation, Spraypolymerisation, inverse Emulsionspolymerisation oder inverse Suspensionspolymerisation. Bevorzugt wird die Lösungspolymerisation in Wasser als Lösemittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818. Die Offenbarungen werden hiermit als Referenz eingeführt und gelten somit als Teil der Offenbarung.

Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Hierzu gehören thermische Initiatoren, Redoxinitiatoren und Photoinitiatoren, deren Aktivierung durch energiereiche Strahlung erfolgt. Die Polymerisationsinitiatoren können dabei in einer Lösung erfindungsgemäßer Monomere gelöst oder dispergiert enthalten sein. Bevorzugt ist der Einsatz wasserlöslicher Initiatoren.

Als thermische Initiatoren kommen sämtliche dem Fachmann bekannte, unter Temperatureinwirkung in Radikale zerfallende Verbindungen in Betracht. Besonders bevorzugt sind dabei thermische Polymerisationsinitiatoren mit einer Halbwertszeit von weniger als 10 Sekunden, darüber hinaus bevorzugt von weniger als 5 Sekunden bei weniger als 180 °C, darüber hinaus bevorzugt bei weniger als 140°C. Dabei sind Peroxide, Hydroperoxide, Wasserstoffperoxid, Persulfate sowie Azoverbindungen besonders bevorzugte thermische Polymerisationsinitiatoren. In manchen Fällen ist es vorteilhaft, Mischungen verschiedener thermischer Polymerisationsinitiatoren zu verwenden. Unter diesen Mischungen sind die aus Wasserstoffperoxid und Natrium- oder Kaliumperoxodisulfat bevorzugt, die in jedem denkbaren Mengenverhältnis eingesetzt werden können. Geeignete organische Peroxide sind vorzugsweise Acetylacetonperoxid, Methylethylketonperoxid, Benzoylperoxid, Lauroylperoxid, Acetylperoxid, Capyrlperoxid, Isopropylperoxydicarbonat, 2-Ethylhexylperoxydicarbonat, t-Butylhydroperoxid, Cumolhydroperoxid, t-Amylperpivalat, t-Butylperpivalat, t-Butylperneohexonat, t-Butylisobutyrat, t-Butylper-2-ethylhexenoat, t-Butylperisononanoat, t-Butylpermaleat, t-Butylperbenzoat, t-Butyl-3,5,5-tri-methylhexanoat und Amylperneodekanoat. Weiterhin sind als thermische Polymerisationsinitiatoren bevorzugt: Azo-Verbindungen, wie Azobisisobutyronitrol, Azobisdimethylvaleronitril, 2,2'-Azobis-(2-amidinopropan)dihydrochlorid, Azo-bis-amidinopropan-dihydrochlord, 2,2'-Azobis-(N,N-dimethylen)isobutyramidin-dihydrochlorid, 2- (Carbamoylazo)- isobutyronitril und 4,4'-Azobis-(4-cyahovaleriansäure). Die genannten Verbindungen werden in üblichen Mengen eingesetzt, vorzugsweise in einem Bereich von 0,01 bis 5, bevorzugt von 0,1 bis 2 Mol-%, jeweils bezogen auf die Menge der zu polymerisierenden Monomere.

Die Redoxinitiatoren enthalten als oxidische Komponente mindestens eine der oben angegebenen Perverbindungen und als reduzierende Komponente vorzugsweise Ascorbinsäure, Glukose, Sorbose, Manose, Ammonium- oder Alkalimetall-hydrogensulfit, - sulfat, -thiosulfat, -hyposulfit oder -sulfid, Metallsalze, wie Eisen-II-ionen oder Silberionen oder Natriumhydroxymethylsulfoxylat. Vorzugsweise wird als reduzierende Komponente des Redoxinitiators Ascorbinsäure oder Natriumpyrosulfit verwendet. Bezogen auf die bei der Polymerisation eingesetzte Menge an Monomeren wird 1×10⁻⁵ bis 1 Mol-% der reduzierenden Komponente des Redoxinitiators und 1×10⁻⁵ bis 5 Mol-% der oxidierenden Komponente des Redoxinitiators eingesetzt. Anstelle der oxidierenden Komponente des Redoxinitiators, oder in Ergänzung zu diesem, können ein oder mehrere, vorzugsweise wasserlösliche, Azoverbindungen verwendet werden.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte α-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)malein- imid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclo-hexanon und 2,6-Bis-(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.

Bevorzugt wird erfindungsgemäß ein Initiatorsystem bestehend aus Wasserstoffperoxid, Natriumperoxodisulfat und Ascorbinsäure eingesetzt. In der Regel wird die Polymerisation mit den Initiatoren in einem Temperaturbereich von 0°C bis 90°C initiiert.

Die Polymerisationsreaktion kann durch einen Initiator oder durch mehrere, zusammenwirkende Initiatoren ausgelöst werden. Weiterhin kann die Polymerisation derart durchgeführt werden, dass man zunächst ein oder mehrere Redoxinitiatoren zusetzt. Im weiteren Polymerisationsverlauf werden dann zusätzlich thermische Initiatoren oder Photoinitiatoren appliziert, wobei im Falle von Photoninitiatoren die Polymerisationsreaktion dann durch die Einwirkung energiereicher Strahlung initiiert wird. Auch die umgekehrte Reihenfolge, also die anfängliche Initiierung der Reaktion mittels energiereicher Strahlung und Photoinitiatoren oder thermischen Initiatoren und eine im weiteren Polymerisationsverlauf erfolgende Initiierung der Polymerisation mittels eines oder mehrerer Redoxinitiatoren ist denkbar.

Um die so erhaltenen Hydrogel-Polymere (VP) in eine partikuläre Form zu überführen, können diese nach ihrer Abtrennung aus der Reaktionsmischung zunächst bei einer Temperatur in einem Bereich von 20 bis 300°C, bevorzugt in einem Bereich von 50 bis 250°C und besonders bevorzugt in einem Bereich von 100 bis 200°C bis hin zu einem Wassergehalt von weniger als 40 Gew.-%, bevorzugt von weniger als 20 Gew.-% und darüber hinaus bevorzugt von weniger als 10 Gew.-%, jeweils bezogen auf das Gesamtgewicht des Hydrogel-Polymers (VP), getrocknet werden. Die Trocknung erfolgt vorzugsweise in dem Fachmann bekannten Öfen oder Trocknern, beispielsweise in Bandtrocknern, Hordentrocknern, Drehrohröfen, Wirbelbetttrocknern, Tellertrocknern, Paddeltrocknern oder Infrarottrocknern.

Gemäß der vorliegenden Erfindung erfolgt das Zerkleinern dabei vorzugsweise durch Trockenmahlen, vorzugsweise durch Trockenmahlen in einer Hammermühle, einer Stiftmühle, einer Kugelmühle oder einer Walzenmühle. In einer weiteren Ausführung der vorliegenden Erfindung kann die Zerkleinerung des Hydrogel-Polymeren auch durch die Kombinationen von mehreren der vorbeschriebenen Mühlen erfolgen.

In einer bevorzugten Ausführungsform der erfindungsgemäßen Verfahren werden als wasserabsorbierende Polymere, Partikel erhalten, die einen Innenbereich und einen den Innenbereich begrenzenden Oberflächenbereich aufweisen. Wobei der Oberflächenbereich eine andere chemische Zusammensetzung als der Innenbereich aufweist oder sich in einer physikalischen Eigenschaft vom Innenbereich unterscheidet. Physikalische Eigenschaften, in denen sich der Innenbereich vom Oberflächenbereich unterscheidet, sind beispielsweise die Ladungsdichte oder der Vernetzungsgrad.

Diese einen Innenbereich und einen den Innenbereich begrenzenden Oberflächenbereich aufweisenden wasserabsorbierenden Polymere sind vorzugsweise dadurch erhältlich, dass oberflächennahe, reaktive Gruppen der Partikel des partikulären Hydrogel-Polymers (VP) nachvernetzt werden. Diese Nachvernetzung kann thermisch, photochemisch oder chemisch erfolgen.

Als Nachvernetzer bevorzugt sind die im Zusammenhang mit den Vernetzern (α3) genannten Verbindungen der Vernetzerklasse II und IV.

Unter diesen Verbindungen sind als Nachvernetzer besonders bevorzugt Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on.

Besonders bevorzugt wird Ethylencarbonat als Nachvernetzer eingesetzt.

Bevorzugte Ausführungsformen der wasserabsorbierenden Polymere sind diejenigen, die durch Vernetzer der folgenden Vernetzerklassen bzw. durch Vernetzer der folgenden Kombinationen von Vernetzerklassen nachvernetzt sind: II, IV und II IV.

Vorzugsweise wird der Nachvernetzer in einer Menge in einem Bereich von 0,01 bis 30 Gew.-%, besonders bevorzugt in einer Menge in einem Bereich von 0,1 bis 20 Gew.-% und darüber hinaus bevorzugt in einer Menge in einem Bereich von 0,3 bis 5 Gew.-%, jeweils bezogen auf das Gewicht der superabsorbierenden Polymere bei der Nachvernetzung eingesetzt.

Es ist ebenfalls bevorzugt, dass die Nachvernetzung dadurch erfolgt, dass ein Lösemittel, umfassend vorzugsweise Wasser, mit Wasser mischbare organische Lösemittel wie etwa Methanol oder Ethanol oder Mischungen aus mindestens zwei davon, sowie den Nachvernetzer mit dem Aussenbereich der Hydrogel-Polymerteilchen bei einer Temperatur in einem Bereich von 30 bis 300°C, besonders bevorzugt in einem Bereich von 100 bis 200°C in Kontakt gebracht werden. Das in Kontakt bringen erfolgt dabei vorzugsweise durch Aufsprühen, der Mischung bestehend aus Nachvernetzer und Lösemittel, auf die Hydrogel-Polymerteilchen und anschließendes Mischen der mit der Mischung in Kontakt gebrachten Hydrogel-Polymerteilchen. Dabei ist der Nachvernetzer in der Mischung vorzugsweise in einer Menge in einem Bereich von 0,01 bis 20 Gew.-%, besonders bevorzugt in einer Menge in einem Bereich von 0,1 bis 10 Gew.-%, bezogen auf das Gesamtgewicht der Mischung, enthalten. Es ist weiterhin bevorzugt, dass in einer Menge in einem Bereich von 0,01 bis 50 Gew.-%, besonders bevorzugt in einer Menge in einem Bereich von 0,1 bis 30 Gew.-%, jeweils bezogen auf das Gewicht der Hydrogel-Polymerteilchen, mit den Hydrogel-Polymerteilchen in Kontakt gebracht wird.

Als Kondensationsreaktionen kommen vorzugsweise die Bildung von Ester-, Amid-, Imid- oder Urethanbindungen in Betracht, wobei die Bildung von Esterbindung bevorzugt ist. Darüber hinaus können dem erfindungsgemäßen Hydrogel-Polymeren und/oder wasserabsorbierenden Polymeren weitere Additive und Effektstoffe zugesetzt werden.

Als Additive sind des weiteren Trennmittel bevorzugt, wie etwa anorganische oder organische pulverförmige Trennmittel. Diese Trennmittel werden vorzugsweise in Mengen in einem Bereich von 0 bis 2 Gew.-%, besonders bevorzugt in einem Bereich von 0,1 bis 1,5 Gew.-%, bezogen auf das Gewicht des Hydrogel-Polymers und/oder des wasserabsorbierenden Polymers, eingesetzt. Bevorzugte Trennmittel sind Holzmehl, Pulp Fasern, pulverförmige Rinde, Cellulosepulver, mineralische Füllstoffe wie Perlit, synthetische Füllstoffe wie Nylonpulver, Rayonpulver, Diatomerde, Bentonit, Kaolin, Zeolithe, Talk, Lehm, Asche, Kohlenstaub, Magnesiumsilikate, Dünger oder Mischungen der Substanzen. Hochdisperse pyrogene Kieselsäure wie sie unter dem Handelsnamen Aerosil von Evonik Degussa vertrieben wird ist bevorzugt.

In einer weiteren bevorzugten Ausführungsform des erfindungsgemäßen Verfahrens erfolgt das in Kontakt bringen der Hydrogel-Polymerteilchen und oder der wasserabsorbierenden Polymerteilchen mit einen Effektstoff, wie z.B. einem Polyzucker, einer polyphenolischen Verbindung wie z.B. hydrolisierbare Tannine oder einer Silizium-Sauerstoff beinhaltenden Verbindung oder einer Mischung von mindestens zwei darauf basierenden Effektstoffen. Der Effektstoff kann sowohl in fester Form (Pulver) als auch in gelöster form mit einem Lösemittel zugegeben werden, wobei die Zugabe des Effektstoffs frühestens nach dem Verfahrensschritt iii) erfolgt. Als Effektstoff wird im Rahmen der vorliegenden Erfindung ein Stoff verstanden, der zur Geruchsinhibierung dient.

Erfindungsgemäß werden hierunter Polyzucker verstanden unter denen der Fachmann solche aus der Gruppe von den geläufigen Stärken und deren Derivate, Cellulosen und deren Derivate, Cyclodextrine versteht. Wobei als Cyclodextrine vorzugsweise α-Cyclodextrin, β-Cyclodextrin, γ-Cyclodextrin oder Mischungen aus diesen Cyclodextrinen verstanden werden.

Als Silizium-Sauerstoff beinhaltende Verbindungen sind Zeolithe bevorzugt. Als Zeolithe können alle dem Fachmann bekannten synthetischen oder natürlichen Zeolithe eingesetzt werden. Bevorzugte natürliche Zeolithe sind Zeolithe aus der Natrolith-Gruppe Harmoton-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabazit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmotom, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS®.

Als Kationen enthalten die in dem erfindungsgemäßen Verfahren eingesetzten Zeolithe vorzugsweise Alkalimetall-Kationen wie Li⁺, Na⁺, K⁺, Rb⁺, Cs⁺ oder Fr⁺ und/oder Erdalkalimetall-Kationen wie Mg²⁺, Ca²⁺, Sr²⁺ oder Ba²⁺.

Als Zeolithe können Zeolithe des sogenannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie beta-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Vorzugsweise werden die Zeolithe als Partikel mit einer mittleren Partikelgröße in einem Bereich von 1 bis 500 µm, besonders bevorzugt in einem Bereich von 2 bis 200 µm und darüber hinaus bevorzugt in einem Bereich von 5 bis 100 µm eingesetzt.

Die Effektstoffe werden in den erfindungsgemäßen Verfahren vorzugsweise in einer Menge in einem Bereich von 0,1 bis 50 Gew.-%, besonders bevorzugt in einem Bereich von 1 bis 40 Gew.-% und darüber hinaus bevorzugt in einer Menge in einem Bereich von 5 bis 30 Gew.-%, jeweils bezogen auf das Gewicht der Hydrogel-Polymerteilchen und oder wasserabsorbierenden Polymerteilchen, eingesetzt.

Als keimhemmende Mittel sind grundsätzlich alle gegen grampositive Bakterien wirksamen Stoffe bevorzugt, wie z. B. 4-Hydroxybenzoesäure und ihre Salze und Ester, N-(4-Chlorphenyl)-N'-(3,4dichlorphenyl)harnstoff, 2,4,4'-Trichlor-2'-hydroxydiphenylether (Triclosan), 4-Chlor-3,5-dimethylphenol, 2,2'- Methylen-bis(6-brom-4-chlorphenol), 3-Methyl-4-(1-methylethyl)phenol, 2-Benzyl-4-chlorphenol, 3-(4- Chlorphenoxy)-1,2-propandiol, 3-Iod-2-propinylbutylcarbamat, Chlorhexidin, 3,4,4'-Trichlorcarbonilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Trüsopropylcitrat, Tributylcitrat und insbesondere Triethylcitrat (Hydagen TM CAT, Cognis GmbH, Düsseldorf/Deutschland). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.
Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, alpha -Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, beta-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien reduzieren durch Beeinflussung der Aktivität der ekkrinen Schweißdrüsen die Schweißbildung, und wirken somit Achselnässe und Körpergeruch entgegen. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium- Zirkonium-Trichlorohydrat, Aluminium-Zirkonium-Tetrachlorohydrat, Aluminium-Zirkoniurn-Pentachlorohydrat und deren Komplexverbindungen z. B. mit Aminosäuren wie Glycin.

Zum Mischen bzw. Besprühen sind alle Vorrichtungen geeignet, die eine homogene Verteilung einer Lösung, Pulver, Suspension oder Dispersion auf oder mit den Hydrogel-Polymerteilchen (VP) oder wasserabsorbierenden Polymeren erlauben. Beispiele sind Lödige Mischer (hergestellt durch die Firma *Gebrüder Lödige Maschinenbau GmbH*), Gericke Multi-Flux Mischer (hergestellt durch die Firma *Gericke* GmbH), DRAIS-Mischer (hergestellt durch die Firma *DRAIS GmbH* Spezialmaschinenfabrik Mannheim), Hosokawa Mischer (*Hosokawa Mokron Co., Ltd*.), Ruberg Mischer (hergestellt durch die Firma *Gebr. Ruberg GmbH* & *CO.KG Nieheim*), Hüttlin Coater (hergestellt durch die Firma *BWI Hüttlin GmbH Steinen*), Fließbetttrockner oder Sprühgranulatoren von AMMAG (hergestellt durch die Firma *AMMAG Gunskirchen, Österreich*) oder Heinen (hergestellt durch die Firma *A. Heinen AG Anlagenbau Varel*), Patterson-Kelly-Mischer, NARA-Schaufelmischer, Schneckenmischer, Tellermischer, Wirbelschichttrockner oder Schugi-Mischer. Für das in Kontakt bringen in einer Wirbelschicht können alle dem Fachmann bekannten und geeignet erscheinenden Wirbelschichtverfahren angewandt werden. Beispielsweise kann ein Wirbelschichtcoater eingesetzt werden.

Einen weiteren Beitrag zur Lösung der eingangs beschriebenen Aufgaben liefert ein Verbund, beinhaltend die erfindungsgemäßen wasserabsorbierenden Polymere oder das Hydrogel-Polymere bzw. die durch die erfindungsgemäßen Verfahren erhältlichen wasserabsorbierenden Polymere oder Hydrogel-Polymere und ein Substrat. Es ist dabei bevorzugt, dass die erfindungsgemäßen wasserabsorbierenden Polymere oder Hydrogel-Polymere und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder Schäume bevorzugt. Weiterhin ist es erfindungsgemäß bevorzugt, dass der Verbund mindestens einen Bereich umfasst, welcher wasserabsorbierenden Polymere oder Hydrogel-Polymere in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm³, vorzugsweise mindestens 0,1 cm³ und am meisten bevorzugt mindestens 0,5 cm³ aufweist.

Einen weiteren Beitrag zur Lösung mindestens einer der Eingangs genannten Aufgaben liefert ein Verfahren zur Herstellung eines Verbundes, wobei die erfindungsgemäßen wasserabsorbierenden Polymere bzw. die durch das erfindungsgemäße Verfahren erhältlichen Superabsorber und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Einen Beitrag zur Lösung mindestens einer der eingangs genannten Aufgaben liefert auch ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

### TESTMETHODEN

Sofern nicht nachfolgend anders angegeben, werden die hierin erfolgten Messungen nach ERT-Verfahren. "ERT"steht für *EDANA Recommended Test* und "EDANA" für *European Disposable and Nonwoven Association.* Alle Testmethoden werden grundsätzlich, wenn nichts anderes angegeben ist, bei einer Umgebungstemperatur von 23±2 °C und einer relativen Luftfeuchte von 50±10 % durchgeführt werden.

### Partikelgrößeverteilung (PSD Particle Size Distribution)

Die Partikelgrößenverteilung der wasserabsorbierenden Polymerpartikel wird analog der von der EDANA empfohlenen Testmethode Nr. WSP 220.3-10 "Particle Size Distribution" bestimmt.

### Zentrifugenretentionskapazität (CRC Centrifuge Retention Capacity)

Die Bestimmung der Zentrifugenretentionskapazität erfolgte gemäß der EDANA (European Disposables and Nonwovens Association) empfohlenen Testmethode Nr. WSP 241.3-10 "Centrifuge retention capacity".

### Bestimmung der "Free Swell Rate" (FSR)

Die Bestimmung der Absorptionsgeschwindigkeit erfolgte über die Messung der sogenannten "*Free Swell Rate* - *FSR*" gemäß dem in der EP-A-0 443 627 auf der Seite 12 beschriebenen Testverfahren.

### Absorption gegen einen Druck von 0,7 psi (AAP)

Die Absorption unter Druck wurde als AAP (Absorption Against Pressure) nach WSP 242.3-10 an der gesamten Kornfraktion bestimmt. Entsprechendwurden0,90g der Prüfsubstanz (abgesiebt zwischen 150 und 850µm) in einen Testzylinder mit 60,0 mm Innendurchmesser und einem Siebboden (400mesh) eingewogen (Konzentration: 0,032 g/cm²) und gleichmäßig verteilt. Auf die Prüfsubstanz wird ein zylindrisches Gewicht 50 g/cm² = 0,7psi) mit einem Außendurchmesser von 59,2 mm gelegt. In eine Kunststoffschale werden Filterplatten gelegt, die mit einem Filterpapier abgedeckt werden. Die Kunststoffschale wird mit 0,9 %iger NaCl-Lösung gefüllt, bis der Flüssigkeitsspiegel mit der Oberkante der Filterplatten abschließt. Anschließend werden die vorbereiteten Messeinheiten auf die Filterplatten gestellt. Nach einer Quellzeit von 60 Minuten werden die Messeinheiten entnommen und das Gewicht entfernt. Die aufgenommene Flüssigkeitsmenge wird gravimetrisch ermittelt und auf 1 Gramm Prüfsubstanz umgerechnet.

### Bestimmung der Permeabilität (saline flow conductivity - SFC)

Die Bestimmung der Permeabilität erfolgt durch die Messung der sogenannten "Saline Flow Conductivity-SFC" gemäß dem in der WO-A-95/26209 beschriebenen Testverfahren. Die Einwaage des Superabsorbermaterials betrug 1,5g anstatt 0,9g.

### Bestimmung "Fixed Height Absorption" (FHA) (0,3 psi, 20 cm)

Die Bestimmung erfolgt durch die Messung der sogenannten "fixed height absorption" (FHA) gemäß dem in der EP 149 34 53 A1 beschriebenen Testverfahren auf der Seite 9 [0078] bis Seite 10 Absatz [0087].

### Bestimmung der Oberflächenspannung (OFS)

Die Bestimmung erfolgt durch die Messung gemäß dem in EP 1 493 453 A1 beschriebenen Testverfahren gemäß Seite 12 Absatz [0105] bis [0111].Es wurde ein Tensiometer der Marke Kruss K11 mit einer Wilhelmyplatte verwendet.

### Bestimmung der extrahierbaren Polyether Anteile

Die Bestimmung der extrahierbaren Anteile der Polyether ist angelehnt an die HPTLC Methode(H-III 14a Stand 30.05.95) Gemeinschaftsarbeiten der DGF 152 Mitteilung. Die Proben wurden gemahlen, mit Methanol im Soxhlet warm extrahiert (1Stunde), eingeengt und danach dünnschichtchromatographisch bestimmt und zwar auf TLC-Platten aus Kieselgel 60 mit den Abmessungen 20x 20 cm. Die Auftragung der Proben erfolgt standardisiert. Als Fließmittel wurde eine Mischung aus Chloroform/Methanol/ Wasser(88/11/1 Volumenprozente) verwendet. Die Derivatisierung erfolgte mit dem Dragendorff s Reagenz. Es wurde ein TLC Scanner Camag CD60 mit einer Messwellenlänge von 520 nm verwendet. Die Auswertung erfolgte über die Peakflächen die ins Verhältnis zu den Peakflächen der Standards gesetzt wurden.

### Bestimmung der "BET Oberfläche"

Die BET Oberfläche der Superabsorber wurde mittels eines "tristar 3020 Kr" Geräts von der Firma Micrömeritics (mit Krypton Gas anstelle von Stickstoff) bestimmt und analog der ISO 9277 gearbeitet. Die Einwaage betrug 4,6 g und wurde über Nacht bei Raumtemperatur und einem Vakuum von 0,02 mbar evakuiert und zurückgewogen. Es erfolgte eine Evakuierung am Analysenport, wobei die Evakuierung über einen Zeitraum von 6 Minutenerfolgt wenn 10mm HG erreicht worden sind. Die Totvolumenbestimmung erfolgte ebenfalls. Die Analysentemperatur betrug 77,1 K. Als p0 wurde 2,32 mmHG (unter Verwendung von Krypton Gas) definiert. Die Ausgleichszeit für das Gleichgewicht wurde mindestens 10 mal gemessen (5 sec, bei einer Änderungsrate von kleiner 0,01%).

### Beispiele

Die nachfolgenden Beispiele dienen der näheren Erläuterung der Erfindung ohne sie jedoch hierauf zu beschränken.

**Tabelle 1**

| **Tensind - Beispiel Nr.** | **Name (supplier)** | **Beschreibung (inkl. Reihenfolge der Blöcke)** | **h(EO)** | **m(PO)** | **R** |
|---|---|---|---|---|---|
| 1 | PE7089 (Evonik Industries) | Allyl-10EO-b-10PO-Acetyl | 10 | 10 | -C(=O)CH₃ |
| 2 | PE7087 (Evonik Industries) | Allyl-10PO-b-10EO-Acetyl | 10 | 10 | -C(=O)CH₃ |
| 3 + | PE7086 (Evonik Industries) | Allyl-10EO-b-10PO-Methyl | 10 | 10 | -CH₃ |
| 4 + | Pluriol ® A111 R (BASF) | Allyl-iEO-b-jPO-Methyl | - | - | -CH₃ |
| 5 | PE7514 (Evonik Industries) | Allyl-6EO-b-6PO-Acetyl | 6 | 6 | -C(=O)CH₃ |
| 6 | PE7316/02 (Evonik Industries) | Allyl-6EO-b-6PO-Hydroxyl | 6 | 6 | -H |
| 7 + | Pluriol ® A23 R (BASF) | Allyl-qEO-b-rPO-Hydroxyl | - | - | -H |
| 8 | PE7065 (Evonik Industries) | Allyl-10PO-b-20EO-Hydroxyl | 20 | 10 | -H |
| 9 + | PE7081 (Evonik Industries) | Allyl-10PO-b-20EO-Methyl | 20 | 10 | -CH₃ |
| 10 + | Natrium Laurylethersulfat (Hansa-Group) | Natrium Laurylethersulfat | - | - | - |

| | | | | | |
|---|---|---|---|---|---|
| + nicht erfindungsgemäßes Tensid | | | | | |

Die nachfolgenden Beispiele, zeigen den synergistischen Effekt auf den FSR-Wert durch die gleichzeitige Verwendung von polymerisierbaren Tensiden und Carbonat. Hierbei wurde eine definierte Partikelgrößenverteilung (PSD) verwendet (150µm bis 710µm). Als Standardmischung wird eine Zusammensetzung von 4 Partikelfraktionen vor der Oberflächennachvernetzung eingesetzt, die sich wie folgt verteilen: 15 Gew.% einer Partikelgröße von 150 µm bis 300µm; 50 Gew.% 300µm bis 500µm; 30 Gew.% 500µm bis 600µm und 5 Gew.% 600µm bis 710µm. Die PSD wird für die Hydrogel-Polymere eingestellt.
In der EP1 701786 B1 wird in den Absätzen [0115 bis 0117] das (Massenmittel der Partikeldurchmesserals D50 definiert. Erfindungsgemäß wird ein Bereich zwischen 300 und 600µm bevorzugt. Besonders bevorzugt ist ein Bereich von 350 und 550µm, und ganz besonders bevorzugt ein Bereich zwischen 400 und 500µm.
Bevorzugt ist ein Blähmittel, bei dem mehr als 35 Gew % der Partikel eine Teilchengröße von 100-300µm aufweisen.

Unter dem Begriff "SX", wie er in der Beschreibung verwendet wird, versteht man die thermische Oberflächennachvernetzung des Vorproduktes (VP). Das Vorprodukt entspricht dem erzeugten Hydrogel-Polymer nach der ersten Trocknung, mit der vorgenannten Kornverteilung.
Grundsätzlich beziehen sich die prozentualen Angaben des Tensides auf die Acrylsäure (wenn nicht anderes erwähnt wird 320 g) und die des Carbonates auf den Ansatz (1000g).

### Beispiel 1

Das Tensid Nr. 1 "Allyl-10EO-b-10PO-Acetyl" aus der Tabelle 1 wurde als Additiv eingesetzt. Die Mengen an eingesetztem Tensid und Carbonat variierten.

Verwendung ohne polymerisierbares Tensid und Soda:

### Beispiel 1 A (Referenz)

In 975,201g einer wässrigen Lösung aus Natriumacrylat mit einem Neutralisationsgrad von 70 mol% (bez. Acrylsäure) sowie einer gesamt Monomerkonzentration von 39,82 % wurden 0,775 g Polyethylenglycol300diacrylat (PEG300DA) (0,2% bez. auf Acrylsäure/Estergehält entsprechend 83%) und 1,639 g Polyethylenglycol-(440)monoallyletheracrylat (PEGMAE-A) (0,4% bez. auf Acrylsäure/Estergehalt entsprechend 78%) als Vernetzer gelöst. Die Monomerenlösung wurde in einem Kunststoff-Polymerisationsgefäß für 30 Minuten mit Stickstoff durchgespült, um den gelösten Sauerstoff zu entfernen. Bei einer Temperatur von 4 °C wurde die Polymerisation durch die aufeinanderfolgende Zugabe von 0,3 g Natriumperoxodisulfat in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht wurde, zerkleinerte man das Gel mit einem Fleischwolf und trocknete 2 h bei 150 °C in einem Umlufttrockenschrank. Das getrocknete Vorprodukt wurde grob zerstoßen, gemahlen und auf die vorbeschriebene Kornverteilung eingestellt.

### Beispiel 1B (2. Referenz)

In 975,443g einer wässrigen Lösung aus Natriumacrylat mit einem Neutralisationsgrad von 70 mol% (bez. Acrylsäure) sowie einer gesamt Monomerkonzentration von 39,81 % wurden 0,697 g Polyethylenglycol300diacrylat (0,18% bez. auf Acrylsäure/Estergehalt=83%) und 1,475 g Polyethylenglycol-(440)monoallyletheracrylat (0,36% bez. auf Acrylsäure/ Estergehaltentsprechend 78%) als Vernetzer gelöst. Die Monomerenlösung wurde in einem Kunststoff-Polymerisationsgefäß für 30 Minuten mit Stickstoff durchgespült, um den gelösten Sauerstoff zu entfernen. Bei einer Temperatur von 4 °C wurde die Polymerisation durch die aufeinanderfolgende Zugabe von 0,3 g Natriumperoxodisulfat in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht war, zerkleinert man das Gel mit einem Fleischwolf und trocknete 2 h bei 150 °C in einem Umlufttrockenschrank. Das getrocknete Vorprodukt wurde grob zerstoßen, gemahlen und auf die vorbeschriebene Kornverteilung eingestellt.

Verwendung ohne polymerisierbares Tensid und mit 0,2% Soda leicht:

### Beispiel 1C

In 973,443g einer wässrigen Lösung aus Natriumacrylat mit einem Neutralisationsgrad von 70 mol% (bez. Acrylsäure) sowie einer gesamt Monomerkonzentration von 39,89 % wurden 0,697 g Polyethylenglycol300diacrylat (0,18% bez. auf Acrylsäure/Estergehalt=83%) und 1,475 g Polyethylenglycol-(440)monoallyletheracrylat (0,36% bez. auf Acrylsäure/Estergehalt entsprechend 78%) als Vernetzer gelöst. Die Monomerenlösung wurde in einem Kunststoff-Polymerisationsgefäß für 30 Minuten mit Stickstoff durchgespült, um den gelösten Sauerstoff zu entfernen. Bei einer Temperatur von 4 °C wurden 2g fein calcinierte Soda (Fa. Solvay) zugesetzt und die Polymerisation durch die aufeinanderfolgende Zugabe von 0,3 g Natriumperoxodisulfat in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht war, zerkleinerte man das Gel mit einem Fleischwolf und trocknete es 2 h bei 150 °C in einem Umlufttrockenschrank. Das getrocknete Vorprodukt wurde grob zerstoßen, gemahlen und auf vorbeschriebene Kornverteilung eingestellt.

Verwendung von 0,3% polymerisierbarem Tensid:

### Beispiel 1D

In 965,601g einer wässrigen Lösung aus Natriumacrylat mit einem Neutralisationsgrad von 70 mol% (bez. Acrylsäure) sowie einer gesamt Monomerkonzentration von 40,22 % wurden 0,775 g Polyethylenglycol300diacrylat (0,2% bez. auf Acrylsäure/Estergehalt=83%) und 1,639 g Polyethylenglycol-(440)monoallyletheracrylat (0,4% bez. auf Acrylsäure/Esterge-halt entsprechend 78%) als Vernetzer gelöst. Dieser Lösung wurden anschließend 9,6g einer 10%igen wässrigen Lösung des Comonomers Nr. 1 "Allyl-10EO-b-10PO-Acetyl" zugegeben und die Monomerenlösung wird in einem Kunststoff-Polymerisationsgefäß für 30 Minuten mit Stickstoff durchgespült, um den gelösten Sauerstoff zu entfernen. Bei einer Temperatur von 4 °C wurde die Polymerisation durch die aufeinanderfolgende Zugabe von 0,3 g Natriumperoxodisulfat in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht worden war, zerkleinerte man das Gel mit einem Fleischwolf und trocknete 2 h bei 150 °C in einem Umlufttrockenschrank. Das getrocknete Vorprodukt wurde grob zerstoßen, gemahlen und auf die vorbeschriebene Kornverteilung eingestellt.

Verwendung von 0,3% polymerisierbarem Tensid und 0,2% Soda leicht:

### Beispiel 1E

In 963,843g einer wässrigen Lösung aus Natriumacrylat mit einem Neutralisationsgrad von 70 mol% (bez. Acrylsäure) sowie einer gesamt Monomerkonzentration von 40,29 % wurden 0,697 g Polyethylenglycol300diacrylat (0,18% bez. auf Acrylsäure/Estergehalt=83%) und 1,475 g Polyethylenglycol-(440)monoallyletheracrylat (0,36% bez. auf Acrylsäure/Estergehalt entsprechend 78%) als Vernetzer gelöst. Dieser Lösung wurden anschließend 9,6g einer 10%igen wässrigen Lösung des Comonomers Nr. 1 "Allyl-10EO-b-10P0-Acetyl" zugegeben und die Monomerenlösung wird in einem Kunststoff-Polymerisationsgefäß für 30 Minuten mit Stickstoff durchgespült, um den gelösten Sauerstoff zu entfernen. Bei einer Temperatur von 4 °C wurden 2g fein calciniertes Soda (Fa. Solvay) zugesetzt und die Polymerisation durch die aufeinanderfolgende Zugabe von 0,3 g Natriumperoxodisulfat in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht war, wurde das Gel mit einem Fleischwolf zerkleinert und 2 h bei 150 °C in einem Umlufttrockenschrank getrocknet. Das getrocknete Vorprodukt wurde grob zerstoßen, gemahlen und auf die vorbeschriebene Kornverteilung eingestellt.

Die Oberflächennachvernetzung der so erhaltenen Vorprodukte ("VP") erfolgte durch die Beschichtung mit einer Lösung bestehend aus Ethylencarbonat/Wasser/Al-lactat/Al-sulfat im Verhältnis 1/3/0,4/0,3% bez. auf 100g Vorprodukt und nachfolgender Erhitzung bei 170°C über einen Zeitraum von 90min. im Trockenschrank.

Die Ergebnisse für die Beispiele 1A-1E sind in der Tabelle 2 zusammengefasst:

**Tabelle 2**

| **Beispiel Nr.** | **Zusatz Surfactant Monomer** | **Zusatz Soda leicht** | **CRC (VP)** | **CRC (SX)** | **FSR (SX)** | **OFS** | **AAP 0,7psi** | **SFC 1.5g** | **FHA** |
|---|---|---|---|---|---|---|---|---|---|
| | | | [g/g] | [g/g] | [g/g/s] | [mN/m] | [g/g] | [units] | [g/g] |
| 1A + | 0% | 0% | 30,8 | 25,9 | 0,29 | 70,1 | 24,8 | 123 | 22,1 |
| 1B + | 0% | 0% | 31,3 | 25,9 | 0,28 | 70,8 | 25,0 | 107 | 22,4 |
| 1C + | 0% | 0,2% | 31,5 | 28,3 | 0,46 | 68,6 | 25,7 | 83 | 23,3 |
| 1D + | 0,3% | 0% | 31,3 | 26,0 | 0,35 | 54,4 | 24,4 | 75 | 20,9 |
| 1E | 0,3% | 0,2% | 31,4 | 26,0 | 0,58 | 54,7 | 24,9 | 91 | 24,1 |

### Beispiel 2

Der Versuchsaufbäu entspricht Beispiel 1 nur dass als Tensid Allyl-10P0-b-10E0-Acetyl (PE7087, Evonik Industries) eingesetzt wird. Die Mengen des eingesetzten Tensides und Carbonates variierten. Die Oberflächennachvernetzung der so erhaltenen Vorprodukte erfolgte durch die Beschichtung mit einer Lösung bestehend aus Ethylencarbonat/Wasser/Allactat/Al-sulfat im Verhältnis 1/3/0,4/0,3% bez. auf 100g Vorprodukt und nachfolgender Erhitzung bei 170°C über einen Zeitraum von 90min. im Trockenschrank.

Die Ergebnisse für die Beispiele 2D und 2E wurden in der Tabelle 3 zusammengefasst:

**Tabelle 3**

| **Beispiel Nr.** | **Zusatz Surfactant Monomer** | **Zusatz Soda leicht** | **CRC (VP)** | **CRC (SX)** | **FSR (SX)** | **OFS** | **AAP 0,7psi** | **SFC 1.5g** | **FHA** |
|---|---|---|---|---|---|---|---|---|---|
| | | | [g/g] | [g/g] | [g/g/s] | [mN/m] | [g/g] | [units] | [g/g] |
| 1A + | 0% | 0% | 30,8* | 25,9 | 0,29 | 70,1 | 24,6 | 123 | 22,1 |
| 1B + | 0% | 0% | 31,3** | 25,9 | 0,28 | 70,8 | 25,0 | 107 | 22,4 |
| 1C + | 0% | 0,2% | 31,5** | 28,3 | 0,46 | 68,6 | 25,7 | 83 | 23,3 |
| 2D + | 0,3% | 0% | 31,6* | 26,1 | 0,36 | 53,5 | 24,6 | 102 | 17,9 |
| 2E | 0,3% | 0,2% | 31,6** | 26,2 | 0,59 | 52,3 | 24,6 | 93 | 23,0 |
| * Vernetzung PEG300DA / PEGMAE-A : 0,2%/0,4% bez. auf Acrylsäure | | | | | | | | | |
| ** Vernetzung PEG300DA / PEGMAE-A : 0,18%/0,36% bez. auf Acrylsäure | | | | | | | | | |

### Beispiel 3

Der Versuchsaufbau entsprach dem Beispiel 1 nur dass als Tensid Allyl-10EO-b-10PO-Methyl (PE7086, Evonik Industries) eingesetzt wird. Die Mengen des eingesetzten Tensides und Carbonates variierten. Die Oberflächennachernetzung der so erhaltenen Vorprodukte erfolgte durch die Beschichtung mit einer Lösung bestehend aus Ethylencarbonat/Wasser/Al-lactat/Al-sulfat im Verhältnis 1/3/0,4/0,3% bez. auf 100g Vorprodukt und nachfolgender Erhitzung bei 170°C über einen Zeitraum von 90min. im Trockenschrank.

Die Ergebnisse für die Beispiele 3D und 3E sind in der Tabelle 4 zusammengefasst:

**Tabelle 4**

| **Beispiel Nr.** | **Zusatz Surfactant Monomer** | **Zusatz Soda leicht** | **CRC (VP)** | **CRC (SX)** | **FSR (SX)** | **OFS** | **AAP 0,7psi** | **SFC 1.5g** | **FHA** |
|---|---|---|---|---|---|---|---|---|---|
| | | | [g/g] | [g/g] | [g/g/s] | [mN/m] | [g/g] | [units] | [g/g] |
| 1A + | 0% | 0% | 30,8* | 25,9 | 0,29 | 70,1 | 24,6 | 123 | 22,1 |
| 1B + | 0% | 0% | 31,3** | 25,9 | 0,28 | 70,8 | 25,0 | 107 | 22,4 |
| 1C + | 0% | 0,2% | 31,5* | 28,3 | 0,46 | 68,6 | 25,7 | 83 | 23,3 |
| 3D + | 0,3% | 0% | 31,4* | 26,7 | 0,30 | 55,2 | 24,5 | 123 | 19,5 |
| 3E + | 0,3% | 0,2% | 32,0** | 27,3 | 0,56 | 55,7 | 25,3 | 76 | 25,1 |
| * Vernetzung PEG300DA / PEGMAE-A : 0,2%/0,4% bez. auf Acrylsäure | | | | | | | | | |
| ** Vernetzung PEG300DA / PEGMAE-A : 0,18%/0,36% bez. auf Acrylsäure | | | | | | | | | |

### Beispiel 4

Der Versuchsaufbau entsprach dem Beispiel 1 nur dass als Additiv Pluriol ^{®} A111 R (BASF) eingesetzt wird. Die Mengen an eingesetztem Tensid und Carbonat variierten.

Verwendung von 0,3% polymerisierbarem Tensid und 0,2% Soda leicht:

### Beispiel 4D

In 963,601g einer wässrigen Lösung aus Natriumacrylat mit einem Neutralisationsgrad von 70 mol% (bez. Acrylsäure) sowie einer gesamt Monomerkonzentration von 40,30 % wurden 0,775 g Polyethylenglycol300diacrylat (0,2% bez. auf Acrylsäure/Estergehalt entsprechend 83%) und 1,639 g Polyethylenglycol-(440)monoallyletheracrylat (0,4% bez. auf Acrylsäure/Estergehalt entsprechend 78%) als Vernetzer gelöst. Dieser Lösung wurden anschließend 9,6g einer 10%igen wässrigen Lösung des Comonomers Nr. 4 Pluriol ^{®} A111 R (Fa. BASF) zugegeben und die Monomerenlösung wird in einem Kunststöff-Polymerisationsgefäß für 30 Minuten mit Stickstoff durchgespült, um den gelösten Sauerstoff zu entfernen. Bei einer Temperatur von 4 °C wurden 2g fein calciniertes Soda (Fa. Solvay) zugesetzt und die Polymerisation durch die aufeinanderfolgende Zugabe von 0,3 g Natriumperoxodisulfat in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht hatte, wurde das Gel mit einem Fleischwolf zerkleinert und 2 h bei 150 °C in einem Umlufttrockenschrank getrocknet. Das getrocknete Vorprodukt wurde grob zerstoßen, gemahlen und abgesiebt.

Die Oberflächennachvernetzung der so erhaltenen Vorprodukte (VP) erfolgte durch die Beschichtung mit einer Lösung bestehend aus Ethylencarbonat/Wasser/Al-lactat/Al-sulfat im Verhältnis 1/3/0,4/0,3% bez. auf 100g Vorprodukt und nachfolgender Erhitzung bei 170°C über einen Zeitraum von 90min. im Trockenschrank.

Die Ergebnisse für die Beispiele 4A-4E sind in der Tabelle 5 zusammengefasst:

**Tabelle 5**

| **Beispiel Nr.** | **Zusatz Surfactant Monomer** | **Zusatz Soda leicht** | **CRC VP** | **CRC (SX)** | **FSR (SX)** | **OFS** | **AAP0, 7psi** | **SEC 1.5g** | **FHA** |
|---|---|---|---|---|---|---|---|---|---|
| | | | [g/g] | [g/g] | [g/g/s] | [mN/m] | [g/g] | [units] | [g/g] |
| 1A + | 0% | 0% | 30,8* | 25,9 | 0,29 | 70,1 | 24,6 | 123 | 22,1 |
| 1C + | 0% | 0,2% | 31,5 | 28,3 | 0,46 | 68,6 | 25,7 | 83 | 23,3 |
| 4C + | 0,3% | 0% | 29,2* | 25,8 | 0,39 | 52,5 | 25,6 | 126 | 22,6 |
| 4D + | 0,3% | 0,2% | 29,1* | 26,4 | 0,54 | 52,1 | 25,6 | 67 | 22,4 |
| 4E + | 0,3% | 0,2% | 32,1** | 27,6 | 0,51 | 51,5 | 26,0 | 43 | 21,2 |
| * Vernetzung PEG300DA / PEGMAE-A : 0,2%/0,4% bez. auf Acrylsäure | | | | | | | | | |
| ** Vernetzung PEG300DA / PEGMAE-A : 0,18%/0,36% bez. auf Acrylsäure | | | | | | | | | |

### Beispiel 5

Der Versuchsaufbau entsprach dem Beispiel 1 nur dass als Tensid Allyl-6EO-b-6PO-Acetyl (PE7514, Evonik Industries) eingesetzt wird. Die Mengen an eingesetztem Tensid und Carbonat variierten.

Verwendung von 0,3% polymerisierbarem Tensid und 0% Soda leicht:

### Beispiel 5D

In 965,843g einer wässrigen Lösung aus Natriumacrylat mit einem Neutralisationsgrad von 70 mol% (bez. Acrylsäure) sowie einer gesamt Monomerkonzentration von 40,21 % wurden 0,697 g Polyethylenglycol300diacrylat (0,18% bez. auf Acrylsäure/Estergehalt entsprechend 83%) und 1,475 g Polyethylenglycol-(440)monoallyletheracrylat (0,36% bez. auf Acrylsäure/Estergehalt entsprechend 78%) als Vernetzer gelöst. Dieser Lösung wurden anschließend 9,6g einer 10%igen wässrigen Lösung des Comonomers Nr. 5 Allyl-6EO-b-6PO-Acetyl zugegeben und die Monomerenlösung wurde in einem Kunststoff-Polymerisationsgefäß für 30 Minuten mit Stickstoff durchgespült, um den gelösten Sauerstoff zu entfernen. Bei einer Temperatur von 4 °C wird die Polymerisation durch die aufeinanderfolgende Zugabe von 0,3 g Natriumperoxodisulfat in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht ist, wird das Gel mit einem Fleischwolf zerkleinert und 2 h bei 150 °C in einem Umlufttrockenschrank getrocknet. Das getrocknete Vorprodukt wird grob zerstoßen, gemahlen und abgesiebt.

Die Oberflächennachvernetzung der so erhaltenen Vorprodukte erfolgt durch die Beschichtung mit einer Lösung bestehend aus Ethylencarbonat/Wasser/Al-lactat/Al-sulfat im Verhältnis 1/3/0,4/0,3% bez. auf 100g Vorprodukt und nachfolgender Erhitzung bei 170°C über einen Zeitraum von 90min. im Trockenschrank.

Die Ergebnisse für die Beispiele 5C-5E sind in der Tabelle 6 zusammengefasst:

**Tabelle 6**

| **Beispiel Nr.** | **Zusatz Surfactant Monomer** | **Zusatz Soda leicht** | **CRC VP** | **CRC (SX)** | **FSR (SX)** | **OFS** | **AAP 1,5g** | **SFC 1.5g** | **FHA** |
|---|---|---|---|---|---|---|---|---|---|
| | | | [g/g] | [g/g] | [g/g/s] | [mN/m] | [units | [units] | [g/g] |
| 1A + | 0% | 0% | 30,8* | 25,9 | 0,29 | 70,1 | 24,6 | 123 | 22,1 |
| 1C + | 0% | 0,2% | 31,5 | 28,3 | 0,46 | 68,6 | 25,7 | 83 | 23,3 |
| 5C | 0,2% | 0,2% | 31,4** | 27,3 | 0,52 | 58,8 | 26,0 | 93 | 20,6 |
| 5D + | 0,3% | 0% | 29,8* | 25,6 | 0,40 | 57,5 | 25,1 | 114 | 23,1 |
| 5E | 0,3% | 0,2% | 30,4** | 26,3 | 0,59 | 57,8 | 26,0 | 74 | 22,3 |
| * Vernetzung PEG300DA / PEGMAE-A : 0,2%/0,4% bez. auf Acrylsäure | | | | | | | | | |
| ** Vernetzung PEG300DA / PEGMAE-A : 0,18%/0,36% bez. auf Acrylsäure | | | | | | | | | |

### Beispiel 6

Der Versuchsaufbau entsprach dem Beispiel 1 nur dass als Tensid Allyl-6EO-b-6POhydroxyl (PE7316/02, Evonik Industries) eingesetzt wurde. Die Mengen an eingesetztem Tensid und Carbonat variierten. Die Oberflächennachvernetzung der so erhaltenen Vorprodukte erfolgt durch die Beschichtung mit einer Lösung bestehend aus Ethylencarbonat/Wasser/Al-lactat/Al-sulfat im Verhältnis 1/3/0,4/0,3% bez. auf 100g Vorprodukt und nachfolgender Erhitzung bei 170°C über einen Zeitraum von 90min. im Trockenschrank.

Die Ergebnisse für die Beispiele 6C-6E sind in der Tabelle 7 zusammengefasst:

**Tabelle 7**

| **Beispiel Nr.** | **Zusatz Surfactant Monomer** | **Zusatz Soda leicht** | **CRC VP** | **CRC (SX)** | **FSR (SX)** | **OFS** | **AAP 7,0psi** | **SFC 1.5g** | **FHA** |
|---|---|---|---|---|---|---|---|---|---|
| | | | [g/g] | [g/g] | [g/g/s] | [mN/m] | [g/g] | [units] | [g/g] |
| 1A + | 0% | 0% | 30,8* | 25,9 | 0,29 | 70,1 | 24,6 | 123 | 22,1 |
| 1C + | 0% | 0,2% | 31,5 | 28,3 | 0,46 | 68,6 | 25,7 | 83 | 23,3 |
| 6C | 0,2% | 0,2% | 30,7* | 26,8 | 0,53 | 60,0 | 25,8 | 74 | 24,6 |
| 6D+ | 0,3% | 0% | 31,1* | 26,5 | 0,37 | 60,8 | 24,8 | 92 | 23,1 |
| 6E | 0,3% | 0,2% | 31,7** | 26,6 | 0,60 | 59,8 | 25,2 | 81 | 25,2 |
| * Vernetzung PEG300DA / PEGMAE-A : 0,2%/0,4% bez. auf Acrylsäure | | | | | | | | | |
| ** Vernetzung PEG300DA / PEGMAE-A : 0,18%/0,36% bez. auf Acrylsäure | | | | | | | | | |

### Beispiel 7

Der Versuchsaufbau entsprach dem Beispiel 1 nur dass als Additiv Pluriol^{®} A23 R (BASF) eingesetzt wurde. Die Mengen an eingesetztem Tensid und Carbonat variierten. Die Oberflächennachvernetzung der so erhaltenen Vorprodukte erfolgt durch die Beschichtung mit einer Lösung bestehend aus EthylencarbonattWasser/Al-lactat/Al-sulfat im Verhältnis 113/0,4/0,3% bez. auf 100g Vorprodukt und nachfolgender Erhitzung bei 170°C über einen Zeitraum von 90min. im Trockenschrank.

Die Ergebnisse für die Beispiele 7C-7F sind in der Tabelle 8 zusammengefasst:

**Tabelle 8**

| **Beispiel Nr.** | **Zusatz Surfactant Monomer** | **Zusatz Soda leicht** | **CRC VP** | **CRC (SX)** | **FSR (SX)** | **OFS** | **AAP 0,7psi** | **SFC 1.5g** | **FHA** |
|---|---|---|---|---|---|---|---|---|---|
| | | | [g/g] | [g/g] | [g/g/s] | [mN/m] | [g/g] | [units] | [g/g] |
| 1A + | 0% | 0% | 30,8* | 25,9 | 0,29 | 70,1 | 24,6 | 123 | 22,1 |
| 1C + | 0% | 0,2% | 31,5 | 28,3 | 0,46 | 68,6 | 25,7 | 83 | 23,3 |
| 7C + | 0,1% | 0,2% | 29,9* | 25,8 | 0,56 | 62,5 | 25,4 | 90 | 24,4 |
| 7D + | 0,3% | 0% | 30,3* | 26,0 | 0,35 | 65,0 | 25,5 | 115 | 23,9 |
| 7E + | 0,3% | 0,2% | 30,7** | 27,9 | 0,56 | 64,0 | 26,4 | 64 | 25,2 |
| 7F + | 0,3% | 0,2% | 30,0* | 26,4 | 0,62 | 63,4 | 25,4 | 77 | 24,6 |
| * Vernetzung PEG300DA / PEGMAE-A : 0,2%/0,4% bez. auf Acrylsäure | | | | | | | | | |
| ** Vernetzung PEG300DA / PEGMAE-A : 0,18%/0,36% bez. auf Acrylsäure | | | | | | | | | |

### Beispiel 8

Der Versuchsaufbau entsprach dem Beispiel 1 nur dass als Tensid Allyl-10PO-b-20EO-Hydroxyl (PE7065, Evonik Industries) eingesetzt wird. Die Mengen an eingesetztem Tensid und Carbonat variierten. Die Oberflächennachvernetzung der so erhaltenen Vorprodukte erfolgt durch die Beschichtung mit einer Lösung bestehend aus Ethylencarbonat/Wasser/Allactat/Al-sulfat im Verhältnis 1/3/0,4/0,3% bez. auf 100g Superabsorber und nachfolgender Erhitzung bei 170°C über einen Zeitraum von 90min. im Trockenschrank.

Die Ergebnisse für die Beispiele 8D-8E sind in der Tabelle 9 zusammengefasst:

**Tabelle 9**

| **Beispiel Nr.** | **Zusatz Surfactant Monomer** | **Zusatz Soda leicht** | **CRD VP** | **CRD (SX)** | **FSR (SX)** | **OFS** | **AAP 0,7psi** | **SFC 1.5g** | **FHA** |
|---|---|---|---|---|---|---|---|---|---|
| | | | [g/g] | [g/g] | [g/g/s] | [mN/m] | [g/g] | [units] | [g/g] |
| 1A + | 0% | 0% | 30,8* | 25,9 | 0,29 | 70,1 | 24,6 | 123 | 22,1 |
| 1B + | 0% | 0% | 31,3** | 25,9 | 0,28 | 70,8 | 25,0 | 107 | 22,4 |
| 1C + | 0% | 0,2% | 31,5** | 28,3 | 0,46 | 68,6 | 25,7 | 83 | 23,3 |
| 8D + | 0,3% | 0% | 33,1* | 29,1 | 0,40 | 55,8 | 25,0 | 80 | 24,4 |
| 8E | 0,3% | 0,2% | 33,0** | 27,9 | 0,65 | 55,2 | 25,7 | 81 | 23,8 |
| * Vernetzung PEG300DA / PEGMAE-A : 0,2%/0,4% bez. auf Acrylsäure | | | | | | | | | |
| ** Vernetzung PEG300DA / PEGMAE-A : 0,18%/0,36% bez. auf Acrylsäure | | | | | | | | | |

### Beispiel 9

Der Versuchsaufbau entsprach dem Beispiel 1 nur dass als Tensid Allyl-10PO-b-20EO-Methyl (PE7081, Evonik Industries) eingesetzt wurde. Die Mengen an eingesetztem Tensid und Carbonat variieren. Die Oberflächennachvernetzung der so erhaltenen Vorprodukte erfolgt durch die Beschichtung mit einer Lösung bestehend aus Ethylencarbonat/Wasser/Allactat/Al-sulfat im Verhältnis 1/3/0,4/0,3% bez. auf 100g Vorprodukt und nachfolgender Erhitzung bei 170°C über einen Zeitraum von 90min. im Trockenschrank.

Die Ergebnisse für die Beispiele 9D-9E sind in der Tabelle 10 zusammengefasst:

**Tabelle 10**

| **Beispiel Nr.** | **Zusatz Surfactant Monomer** | **Zusatz Soda leicht** | **CRC VP** | **CRC (SX)** | **FSR (SX)** | **OFS** | **AAP 0,7psi** | **SFC 1.5g** | **FHA** |
|---|---|---|---|---|---|---|---|---|---|
| | | | [g/g] | [g/g] | [g/g/s] | [mN/m] | [g/g] | [units] | [g/g] |
| 1A + | 0% | 0% | 30,8* | 25,9 | 0,29 | 70,1 | 24,6 | 123 | 22,1 |
| 1B + | 0% | 0% | 31,3** | 25,9 | 0,28 | 70,8 | 25,0 | 107 | 22,4 |
| 1C + | 0% | 0,2% | 31,5** | 28,3 | 0,46 | 68,6 | 25,7 | 83 | 23,3 |
| 9D + | 0,3% | 0% | 31,3* | 28,0 | 0,40 | 55,8 | 24,9 | 102 | 24,3 |
| 9E + | 0,3% | 0,2% | 32,2** | 28,4 | 0,55 | 56,1 | 25,3 | 90 | 24,7 |
| * Vernetzung PEG300DA / PEGMAE-A : 0,2%/0,4% bez. auf Acrylsäure | | | | | | | | | |
| ** Vernetzung PEG300DA / PEGMAE-A : 0,18%/0,36% bez. auf Acrylsäure | | | | | | | | | |

### Beispiel 10 (Vergleichsbeispiel)

Der Versuchsaufbau entspricht Beispiel 1 nur dass als Tensid Natrium Laurylethersulfat (Hansa-Group AG, Duisburg) eingesetzt wurde. NaLES ist kein einpolymerisiebares Tensid. Die Mengen an eingesetztem Tensid und Carbonat variierten.

Die Oberflächennachvernetzung der so erhaltenen Vorprodukte erfolgt durch die Beschichtung mit einer Lösung bestehend aus Ethylencarbonat/Wasser/Al-lactat/Al-sulfat im Verhältnis 1/3/0,4/0,3% bez. auf 100g Vorprodukt und nachfolgender Erhitzung bei 170°C über einen Zeitraum von 90min. im Trockenschrank.

Die Ergebnisse für die Beispiele 10D-10E sind in der Tabelle 11 zusammengefasst: Tabelle 11

| Beispiel Nr | Zusatz Surfactant Monome | Zusatz Soda leicht | CRC VP | CRC (SX) | FSR (SX) | OFS | AAP 0,7psi | SFC 15g | FHA |
|---|---|---|---|---|---|---|---|---|---|
| | | | [g/g] | [g/g] | [g/g/s] | [mN/m] | [g/g] | [units] | [g/g] |
| 1A | 0% | 0% | 30,8* | 25,9 | 0,29 | 70,1 | 24,6 | 123 | 22,1 |
| 1B | 0% | 0% | 31,3** | 25,9 | 0,28 | 70,8 | 25,0 | 107 | 22,4 |
| 1C | 0% | 0,2% | 31,5** | 28,3 | 0,46 | 68,6 | 25,7 | 83 | 23,3 |
| 10D | 0,3% | 0% | 31,6* | 28,3 | 0,42 | 38,0 | 24,9 | 68 | 16,2 |
| 10E | 0,3% | 0,2% | 32,8** | 28,2 | 0,57 | 37,5 | 25,2 | 62 | 16,3 |
| * Vernetzung PEG300DA / PEGMAE-A : 0,2%/0,4% bez. auf Acrylsäure | | | | | | | | | |
| ** Vernetzung PEG300DA / PEGMAE-A : 0,16%/0,36% bez. auf Acrylsäure | | | | | | | | | |

Bei dem nicht einpolymerisierbaren Tensid zeigte sich eine erhebliche Verschlechterung des FHA-Wertes.

Die OFS-Werte der wasserabsorbierenden Polymere gemäß den vorstehend beschriebenen erfindungsgemäß Beispielen liegen über 50 mN/m, bevorzugt über 55 mN/m, besonders bevorzugt über 60 mN/m und am meisten bevorzugt über 62 mN/m. Der OFS-Wert darf einen Wert von 68 nM/m nicht überschreiten. Vorteilhafterweise wird hierdurch der Rewet-Wert (z.B. Backsheet rewet) in den Windeln minimiert und die Kapillarität des Superabsorbers im Absorbent Core der Windel eingehalten, die somit hohen FHA Werten entsprechen. Durch die Verwendung von kurzen EO und PO Blöcken in den tensidischen Comonomeren wird eine starke Erniedrigung der OFS-Werte vermieden.

Vorteilhafterweise führt die gleichzeitige Verwendung von den tensidischen Comonomeren und Carbonat zu einer synergistischen Steigerung der Werte des FSR und des FHA. Erfindungsgemäß zeigt sich im Besonderen der vorgenannte Effekt bei wasserabsorbierenden Polymerendie einen OFS-Wert, größer als 60 aufweisen. Die erfindungsgemäßen wasserabsorbierenden Polymeren zeigen zusätzlich gute Parametereigenschaften bezüglich der CRC-, SFC-, und AAP-Werte.

Gemäß der nachfolgenden Tabelle konnte gezeigt werden, dass erfindungsgemäß der Einbau der copolymerisierbaren Tenside ("Surfactant Monomeren", SM) in das Netzwerk des Hydrogel-Polymers erfolgte. Die extrahierbaren Anteile sind in der letzen Spalte der Tabelle wiedergegeben. Hierbei wurde die Menge an extrahierbaren Polyetheranteilen bezogen auf die eingesetzte Gesamtmenge an copolymerisierbaren Tensiden (SM) für die Polymerisation in % untersucht. Erfindungsgemäß.wurden grundsätzlich weniger als 10% extrahierbare Tenside/Surfactant-Monomere nachgewiesen. Hierdurch konnte nachgewiesen werden, dass das Tensid in die Polymermatrix des Superabsorbers eingebaut wurde.

| **Beispiel Nr.** | **Extrahierbarer Polyether-Antell (ppm)** | **% bezogen auf eingesetzte polyether-Menge** |
|---|---|---|
| **1A** | <10ppm | - |
| **1D** | 60 | 2,4% |
| **5D** | 150 | 6% |
| **7D** | 85 | 3,4% |
| **8D** | 90 | 3,6% |
| **9D** | 70 | 2,8% |

Aus den Messungen der BET-Oberfläche konnte überraschenderweise nachgewiesen werden, dass die erfindungsgemäßen Hydrogel-Polymere eine um bis zu 18 % erhöhte BET-Oberfläche aufweisen, als die Referenz hydrogel-Polymere ohne Tenside und Soda.

Ergebnisse der BET Messungen:

| **Beispiel Nr** | **Zusatz Surfactant Monomer** | **Zusatz Soda leicht** | **BET [m**²**/g]** | **% Steigerung im Vergleich zu Referenz** |
|---|---|---|---|---|
| 1B | 0% | 0% | 0,0296 | Referenz |
| 4B | 0% | 0,2% | 0,0320 | +8% |
| 7E | 0,3% | 0,2% | 0,0348 | +18% |

## Patentansprüche

1. Ein Verfahren zur Herstellung einer wasserabsorbierenden Polymerzusammensetzung, umfassend die Verfahrenschritte
(i) Mischen von
(α1) 0,1 bis 99,999 Gew.-%, bevorzugt 20 bis 98,99 Gew.-% und besonders bevorzugt 30 bis 98,95 Gew.-% polymerisierbaren, ethylenisch ungesättigten, säuregruppenhaltigen Monomeren oder deren Salze, oder deren Mischungen, wobei das ethylenisch ungesättigte, säuregruppenhaltige Monomere Acrylsäure ist,
(α2) 0 bis 70 Gew.-%, bevorzugt 1 bis 60 Gew.-% und besonders bevorzugt 1 bis 40 Gew.-% polymerisierbaren, ethylenisch ungesättigten, mit (α1) copolymerisierbaren Monomeren,
(α3) 0,001 bis 10 Gew.-%, bevorzugt 0,01 bis 7 Gew.-% und besonders bevorzugt 0,05 bis 5 Gew.-% eines oder mehrerer Vernetzer,
(α4) 0 bis 30 Gew.-%, bevorzugt 1 bis 20 Gew.-% und besonders bevorzugt 5 bis 10 Gew.-% wasserlöslichen Polymeren, sowie
(α5) 0 bis 20 Gew.-%, bevorzugt 0,01 bis 7 Gew.-% und besonders bevorzugt 0,05 bis 5 Gew.-% eines oder mehrerer Hilfsstoffe, wobei die Summe der Gewichtsmengen (α1) bis (α5) 100 Gew.-% beträgt,
(ii) radikalische Polymerisation unter Vernetzung, um ein wasserunlösliches, wässriges unbehandeltes Hydrogel-Polymer zu bilden,
(iii) Trocknen des Hydrogel-Polymers,
(iv) Mahlen und Absieben des Hydrogel -Polymers,
(V) Oberflächennachvernetzung des gemahlenen und gesiebten Hydrogel-Polymers und
(vi) Trocknung und Konfektionierung des wasserabsorbierenden Polymers,
wobei
der wässrigen Monomerlösung, vor der Zugabe des Initiators und dem Start der radikalischen Polymerisation, 0,01 bis 5 Gew.-%, bevorzugt 0,02 bis 2 Gew.-% und besonders bevorzugt 0,07 bis 1 Gew.-% mindestens eines Tensides, bezogen auf die Acrylsäure, aus der Gruppe der nicht ionischen, ionischen oder amphoteren Tenside, die mit den unter (α1) genannten Monomeren copolymerisierbar und ausgewählt aus der Gruppe an Verbindungen der Struktur R¹⁻(EO)ₙ-block-(PO)ₘ-R² sind, wobei R¹ oder R² einen, -OH-, Acetyl- oder Allyl-Rest bedeuten und n = 2 bis 20 und m = 2 bis 20 ist,
und 0,01 bis 5 Gew.-%, bevorzugt 0,02 bis 2 Gew.-% und besonders bevorzugt 0,07 bis 1 Gew.-% eines Blähmittels mit einer Partikelgröße von 10 µm bis 900 µm zugesetzt werden, bezogen auf das wasserabsorbierende Polymer,
und wobei R¹ oder R² einem Allyl-Rest und der andere Rest einem Acetyl-, oder -OH-Rest entspricht.

2. Verfahren zur Herstellung eines Hydrogel-Polymers, umfassend die Verfahrenschritte
(i) Mischen von
(α1) 0,1 bis 99,999 Gew.-%, bevorzugt 20 bis 98,99 Gew.-% und besonders bevorzugt 30 bis 98,95 Gew.-% polymerisierbaren, ethylenisch ungesättigten, säuregruppenhaltigen Monomeren oder deren Salze oder polymerisierbaren, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
(α2) 0 bis 70 Gew.-%, bevorzugt 1 bis 60 Gew.-% und besonders bevorzugt 1 bis 40 Gew.-% polymerisierbaren, ethylenisch ungesättigten, mit (α1) copolymerisierbaren Monomeren,
(α3) 0,001 bis 10 Gew.-%, bevorzugt 0,01 bis 7 Gew.-% und besonders bevorzugt 0,05 bis 5 Gew.-% eines oder mehrerer Vernetzer,
(α4) 0 bis 30 Gew.-%, bevorzugt 1 bis 20 Gew.-% und besonders bevorzugt 5 bis 10 Gew.-% wasserlöslichen Polymeren,
(α5) 0 bis 20 Gew.-%, bevorzugt 0,01 bis 7 Gew.-% und besonders bevorzugt 0,05 bis 5 Gew.-% eines oder mehrerer Hilfsstoffe, wobei die Summe der Gewichtsmengen (α1) bis (α5) 100 Gew.-%,
(ii) radikalische Polymerisation unter Vernetzung, um ein wasserunlösliches, wässriges unbehandeltes Hydrogel-Polymer zu bilden,
(iii) Trocknen des Hydrogel-Polymers,
(iv) Mahlen und Absieben des Hydrogel-Polymers,
wobei
der wässrigen Monomerlösung, vor der Zugabe des Initiators und dem Start der radikalischen Polymerisation, 0,01 bis 5 Gew.-%, bevorzugt 0,02 bis 2 Gew.-% und besonders bevorzugt 0,07 bis 1 Gew.-% mindestens eines Tensides aus der Gruppe der nicht ionischen, ionischen oder amphoteren Tenside, die mit den unter (α1) genannten Monomeren copolymerisierbar und ausgewählt aus der Gruppe an Verbindungen der Struktur R¹⁻(EO)ₙ-block-(PO)ₘ-R² sind, wobei R¹ oder R² einen -OH-, Acetyl- oder Allyl-Rest bedeuten und n = 2 bis 20 und m = 2 bis 20 ist,
und 0,01 bis 5 Gew.-%, bevorzugt 0,02 bis 2 Gew. % und besonders bevorzugt 0,07 bis 1 Gew.-% eines Blähmittels mit einer Partikelgröße von 10 µm bis 900µm zugesetzt werden, bezogen auf das Hydrogel- Polymere,
und wobei R¹ oder R² einem Allyl-Rest und der andere Rest einem Acetyl-, oder -OH-Rest entspricht.

3. Verfahren nach einem der vorhergehenden Ansprüche, **dadurch gekennzeichnet, dass** das Tensid bevorzugt aus der Gruppe der nicht ionischen Tenside ausgewählt ist .

4. Verfahren nach einem der vorhergehenden Ansprüche, **dadurch gekennzeichnet, dass** das Tensid ausgewählt ist aus der Gruppe R¹-(EO)ₙ-block-(PO)ₘ- R² wobei n bevorzugt 4 bis 12 und m bevorzugt 3 bis 12 bedeutet.

5. Verfahren nach einem der vorhergehenden Ansprüche, **dadurch gekennzeichnet, dass** das Tensid aus der Gruppe R¹-(EO)ₙ-block-(PO)ₘ- R² wobei n besonders bevorzugt 5 bis 8 und m besonders bevorzugt 4 bis 7 bedeutet.

6. Verfahren nach einem der vorhergehenden Ansprüche, **dadurch gekennzeichnet dass** für n und m in der Formel R¹-(EO)ₙ-block-(PO)ₘ- R² besonders bevorzugt 2 ≤ m ≤ n ≤ 20 gilt.

7. Verfahren nach einem der vorhergehenden Ansprüche, **dadurch gekennzeichnet, dass** die Reste R¹ oder R² Allyl oder Acetyl und R¹ und/oder R² einen Methyl-, Ethyl-, n-Propyl-, iso-Propyl-, n-Butyl-, iso-Butyl-, n-Pentyl-, 2-Methyl-Butyl-, 2,2-Dimethylpropyl-, -OH-, Acetyl- oder Allyl-Rest darstellen.

8. Verfahren nach einem der vorhergehenden Ansprüche, **dadurch gekennzeichnet, dass** das Tensid und das Blähmittel gemeinsam der Monomerlösung zugesetzt werden.

9. Verfahren nach einem der vorhergehenden Ansprüche, **dadurch gekennzeichnet, dass** die Blähmittel aus einem Pulver aus anorganischen Partikeln bestehen.

10. Verfahren nach einem der vorhergehenden Ansprüche, **dadurch gekennzeichnet, dass** die Blähmittel aus Sodapartikeln bestehen.

11. Verfahren nach einem der vorhergehenden Ansprüche, **dadurch gekennzeichnet, dass** das Blähmittel eine bevorzugte Partikelgröße von 10 µm bis 900 µm, bevorzugt 50 µm bis 500 µm und besonders bevorzugt 100 µm bis 450 µm aufweist.

12. Verfahren nach einem der vorhergehenden Ansprüche, **dadurch gekennzeichnet, dass** mehr als 35 Gew.-% der Blähmittel eine Teilchengröße von 100-300µm aufweisen.

13. Verfahren nach einem der vorhergehenden Ansprüche, **dadurch gekennzeichnet, dass** die FSR im Bereich von 0,3 bis 0,65 bevorzugt 0,35 bis 0,60 beträgt.

14. Verfahren nach einem der vorhergehenden Ansprüche, **dadurch gekennzeichnet, dass** die Oberflächenspannung (OFS) im Bereich von über 50 mN/m, bevorzugt über 55 mN/m, besonders bevorzugt über 60 mN/m und am meisten bevorzugt über 62 mN/m beträgt

15. Verfahren nach einem der vorhergehenden Ansprüche, **dadurch gekennzeichnet, dass** die Permeabilität als SFC (1,5g) im Bereich von 30 bis 200, bevorzugt 50 bis 180, besonders bevorzugt von 70 bis 150 beträgt.

16. Wasserabsorbierendes Polymer erhältlich nach einem der Ansprüche 1 bis 15.

17. Verbund, beinhaltend ein wasserabsorbierendes Polymer nach einem der vorhergehenden Ansprüche.

18. Verfahren zur Herstellung eines Verbundes, wobei ein wasserabsorbierendes Polymer nach einem der vorhergehenden Ansprüche und ein Hilfsmittel miteinander in Kontakt gebracht werden.

19. Verbund, erhältlich nach einem Verfahren gemäß Anspruch 18.

## Claims

1. Process for producing a water-absorbing polymer composition, comprising the process steps of
(i) mixing
(α1) 0.1 to 99.999% by weight, preferably 20 to 98.99% by weight and more preferably 30 to 98.95% by weight of polymerizable, ethylenically unsaturated monomers containing acid groups, or salts thereof, or mixtures thereof, where the ethylenically unsaturated monomer containing acid groups is acrylic acid,
(α2) 0 to 70% by weight, preferably 1 to 60% by weight and more preferably 1 to 40% by weight of polymerizable, ethylenically unsaturated monomers copolymerizable with (α1),
(α3) 0.001 to 10% by weight, preferably 0.01 to 7% by weight and more preferably 0.05 to 5% by weight of one or more crosslinkers,
(α4) 0 to 30% by weight, preferably 1 to 20% by weight and more preferably 5 to 10% by weight of water-soluble polymers, and
(α5) 0 to 20% by weight, preferably 0.01 to 7% by weight and more preferably 0.05 to 5% by weight of one or more assistants, where the sum of the weights of (α1) to (α5) is 100% by weight,
(ii) free-radical polymerization with crosslinking to form a water-insoluble, aqueous untreated hydrogel polymer,
(iii) drying the hydrogel polymer,
(iv) grinding and sieving the hydrogel polymer to size,
(v) surface postcrosslinking the ground and sieved hydrogel polymer
and
(vi) drying and finishing the water-absorbing polymer,
wherein
the aqueous monomer solution, prior to the addition of the initiator and the commencement of free-radical polymerization, is admixed with 0.01 to 5% by weight, preferably 0.02 to 2% by weight and more preferably 0.07 to 1% by weight of at least one surfactant, based on the acrylic acid, from the group of the nonionic, ionic or amphoteric surfactants which are copolymerizable with the monomers specified in (α1) and are selected from the group of compounds of the structure R¹-(EO)ₙ-block-(PO)ₘ-R² where R¹ or R² is a -OH, acetyl or allyl radical and n = 2 to 20 and m = 2 to 20,
and 0.01 to 5% by weight, preferably 0.02 to 2% by weight and more preferably 0.07 to 1% by weight of a blowing agent having a particle size of 10 µm to 900 µm, based on the water-absorbing polymer, and wherein R¹ or R² is an allyl radical and the other radical is an acetyl or -OH radical.

2. Process for producing a hydrogel polymer, comprising the process steps of
(i) mixing
(α1) 0.1 to 99.999% by weight, preferably 20 to 98.99% by weight and more preferably 30 to 98.95% by weight of polymerizable, ethylenically unsaturated monomers containing acid groups, or salts thereof, or polymerizable, ethylenically unsaturated monomers including a protonated or quaternized nitrogen, or mixtures thereof, particular preference being given to mixtures including at least ethylenically unsaturated monomers containing acid groups, preferably acrylic acid,
(α2) 0 to 70% by weight, preferably 1 to 60% by weight and more preferably 1 to 40% by weight of polymerizable, ethylenically unsaturated monomers copolymerizable with (α1),
(α3) 0.001 to 10% by weight, preferably 0.01 to 7% by weight and more preferably 0.05 to 5% by weight of one or more crosslinkers,
(α4) 0 to 30% by weight, preferably 1 to 20% by weight and more preferably 5 to 10% by weight of water-soluble polymers,
(α5) 0 to 20% by weight, preferably 0.01 to 7% by weight and more preferably 0.05 to 5% by weight of one or more assistants, where the sum of the weights of (α1) to (α5) is 100% by weight,
(ii) free-radical polymerization with crosslinking to form a water-insoluble, aqueous untreated hydrogel polymer,
(iii) drying the hydrogel polymer,
(iv) grinding and sieving the hydrogel polymer to size,
wherein
the aqueous monomer solution, prior to the addition of the initiator and the commencement of free-radical polymerization, is admixed with 0.01 to 5% by weight, preferably 0.02 to 2% by weight and more preferably 0.07 to 1% by weight of at least one surfactant from the group of the nonionic, ionic or amphoteric surfactants which are copolymerizable with the monomers specified in (α1) and are selected from the group of compounds of the structure R¹-(EO)ₙ-block-(PO)ₘ-R² where R¹ or R² is a -OH, acetyl or allyl radical and n = 2 to 20 and m = 2 to 20,
and 0.01 to 5% by weight, preferably 0.02 to 2% by weight and more preferably 0.07 to 1% by weight of a blowing agent having a particle size of 10 µm to 900 µm, based on the hydrogel polymer, and wherein R¹ or R² is an allyl radical and the other radical is an acetyl or -OH radical.

3. Process according to either of the preceding claims, **characterized in that** the surfactant is preferably selected from the group of the nonionic surfactants.

4. Process according to any of the preceding claims, **characterized in that** the surfactant is selected from the group of R¹-(EO)ₙ-block-(PO)ₘ-R² where n is preferably 4 to 12 and m is preferably 3 to 12.

5. Process according to any of the preceding claims, **characterized in that** the surfactant from the group of R¹-(EO)ₙ-block-(PO)ₘ-R² where n is more preferably 5 to 8 and m is more preferably 4 to 7.

6. Process according to any of the preceding claims, **characterized in that**, for n and m in the formula R¹-(EO)ₙ-block-(PO)ₘ-R², more preferably,
2≤m≤n≤20.

7. Process according to any of the preceding claims, **characterized in that** the R¹ or R² radicals are allyl or acetyl and R¹ and/or R² are a methyl, ethyl, n-propyl, isopropyl, n-butyl, isobutyl, n-pentyl, 2-methylbutyl, 2,2-dimethylpropyl, -OH, acetyl or allyl radical.

8. Process according to any of the preceding claims, **characterized in that** the surfactant and the blowing agent are added together to the monomer solution.

9. Process according to either of the preceding claims, **characterized in that** the blowing agents consist of a powder of inorganic particles.

10. Process according to any of the preceding claims, **characterized in that** the blowing agents consist of sodium carbonate particles.

11. Process according to any of the preceding claims, **characterized in that** the blowing agent has a preferred particle size of 10 µm to 900 µm, preferably 50 µm to 500 µm and more preferably 100 µm to 450 µm.

12. Process according to any of the preceding claims, **characterized in that** more than 35% by weight of the blowing agents have a particle size of 100-300 µm.

13. Process according to any of the preceding claims, **characterized in that** the FSR is in the range from 0.3 to 0.65, preferably 0.35 to 0.60.

14. Process according to any of the preceding claims, **characterized in that** the surface tension (ST) is in the range above 50 mN/m, preferably above 55 mN/m, more preferably above 60 mN/m and most preferably above 62 mN/m.

15. Process according to any of the preceding claims, **characterized in that** the permeability, as the SFC (1.5g), is in the range from 30 to 200, preferably 50 to 180, more preferably from 70 to 150.

16. Water-absorbing polymer obtainable according to any of Claims 1 to 15.

17. Composite including a water-absorbing polymer according to any of the preceding claims.

18. Process for producing a composite, wherein a water-absorbing polymer according to any of the preceding claims and an assistant are contacted with one another.

19. Composite obtainable by a process according to Claim 18.

## Revendications

1. Procédé pour la préparation d'une composition de polymère absorbant l'eau, comprenant les étapes de procédé de
(i) mélange de
(α1) 0,1 à 99,999% en poids, de préférence 20 à 98,99% en poids et de manière particulièrement préférée 30 à 98,95% en poids de monomères polymérisables, éthyléniquement insaturés, contenant des groupes acides ou leurs sels ou leurs mélanges, le monomère éthyléniquement insaturé, contenant des groupes acides, étant l'acide acrylique,
(α2) 0 à 70% en poids, de préférence 1 à 60% en poids et de manière particulièrement préférée 1 à 40% en poids de monomères polymérisables, éthyléniquement insaturés, copolymérisables avec (α1),
(α3) 0,001 à 10% en poids, de préférence 0,01 à 7% en poids et de manière particulièrement préférée 0,05 à 5% en poids d'un ou de plusieurs réticulants,
(α4) 0 à 30% en poids, de préférence 1 à 20% en poids et de manière particulièrement préférée 5 à 10% en poids de polymères solubles dans l'eau, ainsi que
(α5) 0 à 20% en poids, de préférence 0,01 à 7% en poids et de manière particulièrement préférée 0,05 à 5% en poids d'un ou de plusieurs adjuvants,
la somme des quantités pondérales (α1) à (α5) étant égale à 100% en poids,
(ii) polymérisation par voie radicalaire avec réticulation, pour former un polymère sous forme d'un hydrogel aqueux, non traité, insoluble dans l'eau,
(iii) séchage du polymère sous forme d'hydrogel,
(iv) broyage et tamisage du polymère sous forme d'hydrogel,
(v) post-réticulation en surface du polymère sous forme d'hydrogel broyé et tamisé et
(vi) séchage et confection du polymère absorbant l'eau,
la solution aqueuse de monomères étant additionnée, avant l'addition de l'initiateur et le démarrage de la polymérisation par voie radicalaire, de 0,01 à 5% en poids, de préférence de 0,02 à 2% en poids et de manière particulièrement préférée de 0,07 à 1% en poids, par rapport à l'acide acrylique, d'au moins un agent tensioactif du groupe des agents tensioactifs non ioniques, ioniques ou amphotères qui sont copolymérisables avec les monomères mentionnés sous (α1) et sont choisis dans le groupe des composés de la structure R¹-(OE)ₙ-bloc-(OP)ₘ-R², R¹ ou R² signifiant un radical -OH, acétyle ou allyle et n = 2 à 20 et m = 2 à 20,
et de 0,01 à 5% en poids, de préférence de 0,02 à 2% en poids et de manière particulièrement préférée de 0,07 à 1% en poids, par rapport au polymère absorbant l'eau, d'un agent gonflant présentant une grosseur de particule de 10 µm à 900 µm, et R¹ ou R² représentant un radical allyle et l'autre radical correspondant à un radical acétyle ou -OH.

2. Procédé pour la préparation d'un polymère sous forme hydrogel, comprenant les étapes de
(i) mélange de
(α1) 0,1 à 99,999% en poids, de préférence 20 à 98,99% en poids et de manière particulièrement préférée 30 à 98,95% en poids de monomères polymérisables, éthyléniquement insaturés, contenant des groupes acides ou leurs sels ou de monomères polymérisables, éthyléniquement insaturés, contenant un azote protoné ou quaternisé ou leurs mélanges, en préférant en particulier des mélanges contenant au moins des monomères éthyléniquement insaturés, contenant des groupes acides, de préférence l'acide acrylique,
(α2) 0 à 70% en poids, de préférence 1 à 60% en poids et de manière particulièrement préférée 1 à 40% en poids de monomères polymérisables, éthyléniquement insaturés, copolymérisables avec (α1),
(α3) 0,001 à 10% en poids, de préférence 0,01 à 7% en poids et de manière particulièrement préférée 0,05 à 5% en poids d'un ou de plusieurs réticulants,
(α4) 0 à 30% en poids, de préférence 1 à 20% en poids et de manière particulièrement préférée 5 à 10% en poids de polymères solubles dans l'eau,
(α5) 0 à 20% en poids, de préférence 0,01 à 7% en poids et de manière particulièrement préférée 0,05 à 5% en poids d'un ou de plusieurs adjuvants,
la somme des quantités pondérales (α1) à (α5) étant égale à 100% en poids,
(ii) polymérisation par voie radicalaire avec réticulation, pour former un polymère sous forme d'un hydrogel aqueux, non traité, insoluble dans l'eau,
(iii) séchage du polymère sous forme d'hydrogel,
(iv) broyage et tamisage du polymère sous forme d'hydrogel
la solution aqueuse de monomères étant additionnée, avant l'addition de l'initiateur et le démarrage de la polymérisation par voie radicalaire, par rapport au polymère sous forme d'hydrogel, de 0,01 à 5% en poids, de préférence de 0,02 à 2% en poids et de manière particulièrement préférée de 0,07 à 1% en poids d'au moins un agent tensioactif du groupe des agents tensioactifs non ioniques, ioniques ou amphotères qui sont copolymérisables avec les monomères mentionnés sous (α1) et sont choisis dans le groupe des composés de la structure R¹-(OE)ₙ-bloc-(OP)ₘ-R², R¹ ou R² signifiant un radical -OH, acétyle ou allyle et n = 2 à 20 et m = 2 à 20,
et de 0,01 à 5% en poids, de préférence de 0,02 à 2% en poids et de manière particulièrement préférée de 0,07 à 1% en poids d'un agent gonflant présentant une grosseur de particule de 10 µm à 900 µm, et R¹ ou R² représentant un radical allyle et l'autre radical correspondant à un radical acétyle ou -OH.

3. Procédé selon l'une quelconque des revendications précédentes, **caractérisé en ce que** l'agent tensioactif est de préférence choisi dans le groupe des agents tensioactifs non ioniques.

4. Procédé selon l'une quelconque des revendications précédentes, **caractérisé en ce que** l'agent tensioactif est choisi dans le groupe formé par R¹-(OE)ₙ-bloc-(OP)ₘ-R², n signifiant de préférence 4 à 12 et m de préférence 3 à 12.

5. Procédé selon l'une quelconque des revendications précédentes, **caractérisé en ce que** l'agent tensioactif dans le groupe formé par R¹-(OE)ₙ-bloc-(OP)ₘ-R², n signifiant de manière particulièrement préférée 5 à 8 et m de manière particulièrement préférée 4 à 7.

6. Procédé selon l'une quelconque des revendications précédentes, **caractérisé en ce que**, dans la formule R¹-(OE)ₙ-bloc-(OP)ₘ-R², de manière particulièrement préférée 2 ≤ m ≤ n ≤ 20.

7. Procédé selon l'une quelconque des revendications précédentes, **caractérisé en ce que** les radicaux R¹ ou R² représentent allyle ou acétyle et R¹ et/ou R² représentent un radical méthyle, éthyle, n-propyle, iso-propyle, n-butyle, iso-butyle, n-pentyle, 2-méthyl-butyle, 2,2-diméthylpropyle, -OH, acétyle ou allyle.

8. Procédé selon l'une quelconque des revendications précédentes, **caractérisé en ce que** l'agent tensioactif et l'agent gonflant sont ajoutés ensemble à la solution de monomères.

9. Procédé selon l'une quelconque des revendications précédentes, **caractérisé en ce que** l'agent gonflant est constitué par une poudre de particules inorganiques.

10. Procédé selon l'une quelconque des revendications précédentes, **caractérisé en ce que** l'agent gonflant est constitué par des particules de soude.

11. Procédé selon l'une quelconque des revendications précédentes, **caractérisé en ce que** l'agent gonflant présente une grosseur de particule préférée de 10 µm à 900 µm, de préférence de 50 µm à 500 µm et de manière particulièrement préférée de 100 µm à 450 µm.

12. Procédé selon l'une quelconque des revendications précédentes, **caractérisé en ce que** plus de 35% en poids de l'agent gonflant présentent une grosseur de particule de 100-300 µm.

13. Procédé selon l'une quelconque des revendications précédentes, **caractérisé en ce que** le taux de gonflement libre ("Free Swell Rate" - FSR) se situe dans la plage de 0,3 à 0,65, de préférence de 0,35 à 0,60.

14. Procédé selon l'une quelconque des revendications précédentes, **caractérisé en ce que** la tension superficielle (TS) se situe dans la plage supérieure à 50 mN/m, de préférence supérieure à 55 mN/m, de manière particulièrement préférée supérieure à 60 mN/m et le plus préférablement supérieure à 62 mN/m.

15. Procédé selon l'une quelconque des revendications précédentes, **caractérisé en ce que** la perméabilité, en tant que conductivité de flux salin ("saline flow conductivity" - SFC) (1,5 g) se situe dans la plage de 30 à 200, de préférence de 50 à 180, de manière particulièrement préférée de 70 à 150.

16. Polymère absorbant l'eau, pouvant être obtenu selon l'une quelconque des revendications 1 à 15.

17. Composite, contenant un polymère absorbant l'eau selon l'une quelconque des revendications précédentes.

18. Procédé pour la préparation d'un composite, un polymère absorbant l'eau selon l'une quelconque des revendications précédentes et un adjuvant étant mis en contact l'un avec l'autre.

19. Composite pouvant être obtenu selon un procédé selon la revendication 18.
